# EUROPEAN PATENT APPLICATION

(11) **EP 4 524 578 A1**
(43) Date of publication of application: **19.03.2025**
(21) Application number: 23803573.7
(22) Date of filing: 10.05.2023
(51) Int. Cl.: G01N 35/08, C12M 1/00, C12M 1/34, C12Q 1/686, G01N 37/00

(54) **MICROFLUIDIC CHIP, PCR DEVICE, AND PCR METHOD**

(30) Priority: 12.05.2022 JP 2022078943
(71) Applicant: Go!Foton, Inc., Tsukuba City, Ibaraki 300-2635 (JP)
(72) Inventor: YABU Hiroki, Tokyo 108-6321 (JP); MOTONO Toshinori, Tokyo 108-6321 (JP); KAWAGUCHI Osamu, Tokyo 108-6321 (JP)
(74) Representative: Algemeen Octrooi- en Merkenbureau B.V.
(86) International application number: PCT/JP2023/017537
(87) International publication number: WO 2023/219098

(57) **Abstract**

A microfluidic chip (10) includes: a substrate (14); a channel (12) that is formed on the substrate (14) and that is for a sample to move; a first air coupling port (24) that is provided at an end of the channel (12); a thermal cycle region that is provided in a part of the channel (12) and that is for applying a thermal cycle to the sample in the channel (12); and a first chamber (32) that is provided between the thermal cycle region and the first air coupling port (24) in the channel 12.

## Description

### TECHNICAL FIELD

The present invention relates to a microfluidic chip, a PCR device, and a PCR method used for polymerase chain reactions (PCR).

### BACKGROUND ART

Genetic testing is widely used for examinations in a wide variety of medical fields, identification of farm products and pathogenic microorganisms, safety assessment for food products, and even for examinations for pathogenic viruses and a variety of infectious diseases. In order to detect a minute amount of DNA with high sensitivity, methods of analyzing the resultant obtained by amplifying a portion of DNA are known. Above all, a method that uses PCR is a remarkable technology where a certain portion of a very small amount of DNA collected from an organism or the like is selectively amplified.

In PCR, a predetermined thermal cycle is applied to a sample in which a biological sample containing DNA and a PCR reagent consisting of primers, enzymes, and the like are mixed so as to cause denaturation, annealing, and elongation reactions to be repeated so that a specific portion of DNA is selectively amplified.

It is a common practice to perform PCR by putting a predetermined amount of a target sample into a PCR tube or a chip such as a microplate (microwell) in which a plurality of holes are formed. However, in recent years, PCR using a reaction processing chip provided with a micro-channel that is formed on a substrate is practiced (e.g. Patent Literature 1). A device including a channel through which a sample subjected to PCR flows is referred to as, for example, a (micro) fluidic chip, a (micro) channel chip, a (micro) fluid device, and a (micro) channel chip device, and is also referred to as a nucleic acid amplification chip (device) with a focus on the functional aspect of amplifying nucleic acids.

### RELATED-ART LITERATURE

### PATENT LITERATURE

Patent Literature 1: JP 2009-232700

### SUMMARY OF INVENTION

### TECHNICAL PROBLEM

In a PCR device using a microfluidic chip, a technology is known in which a liquid sample containing a portion of the nucleic acid to be amplified is subjected to a thermal cycle by repeatedly moving the sample in a reciprocating motion within a channel having a width and depth of several tens of µm to several mm. In such a PCR device, the sample repeatedly moves in a reciprocating motion within a thermal cycle region that includes a region set to multiple temperatures required for PCR. The driving force for moving the sample is obtained by blowing air or applying pressure from an air coupling port provided at the end of the channel. In this case, the air blowing and pressure application from the air coupling port are controlled such that the sample stops at a predetermined location in the thermal cycle region. At this time, the output of a pump whose output port is connected to the air coupling port is controlled. The moving speed of the sample inside the channel is high, and the time it takes to move in a reciprocating motion within the thermal cycle region is short. Further, depending on the characteristics of the sample, the viscosity of the liquid sample may change during PCR due to amplification of nucleic acids contained in the sample. For these reasons, it may be difficult to stop the sample at a predetermined position within the channel.

In particular, if the moving sample does not stop at the intended position and goes too far (overruns) the intended stop position, there is a possibility that a portion of the sample may enter an output nozzle of the pump connected to the microfluidic chip, for example. This can cause contamination of the device's liquid delivery system, which can affect the PCR of the next sample. In addition, if a filter for preventing contamination is attached to the microfluidic chip, a portion of the sample may reach the filter and become wet due to an erroneous stop or overrun of the sample.

In this background, a purpose of the present invention is to provide a microfluidic chip capable of preventing various problems that occur when a sample overruns an intended stop position (including a target stop position).

### SOLUTION TO PROBLEM

A microfluidic chip according to one embodiment of the present invention includes: a substrate; a channel that is formed on the substrate and that is for a sample to move; an air coupling port that is provided at an end of the channel; a thermal cycle region that is provided in a part of the channel and that is for applying a thermal cycle to the sample in the channel; and a chamber that is provided between the thermal cycle region and the air coupling port in the channel.

Another embodiment of the present invention relates to a PCR device. This device includes: the microfluidic chip described above; a temperature control system that is for adjusting the temperature of the thermal cycle region; and a liquid feeding system that moves and stops a sample in the channel.

Still another embodiment of the present invention relates to a PCR method. This method includes: introducing a sample into the microfluidic chip described above; adjusting the temperature of the thermal cycle region; and applying a thermal cycle to the sample by moving and stopping the sample in the channel.

### ADVANTAGEOUS EFFECTS OF INVENTION

According to the present invention, a microfluidic chip can be provided that is capable of preventing various problems that occur when a sample overruns an intended stop position.

### BRIEF DESCRIPTION OF DRAWINGS

[Fig. 1] Fig. 1 is a perspective view showing the first surface side of a microfluidic chip according to an embodiment of the present invention.
[Fig. 2] Fig. 2 is a perspective view showing the second surface side of the microfluidic chip.
[Fig. 3] Fig. 3 is a plan view of the first surface of a substrate provided in the microfluidic chip.
[Fig. 4] Fig. 4 is a plan view of the second surface of the substrate provided in the microfluidic chip.
[Fig. 5] Fig. 5 is a conceptual diagram for explaining a cross-sectional structure of the microfluidic chip.
[Fig. 6] Fig. 6 is a schematic diagram for explaining one embodiment of a PCR device for performing a thermal cycle and PCR in which a microfluidic chip can be used.
[Figs. 7] Figs. 7A and 7B are diagrams for explaining the shape of a channel in the substrate provided in the microfluidic chip.
[Fig. 8] Fig. 8 is a diagram showing an exemplary variation of a meandering channel.
[Fig. 9] Fig. 9 is a plan view of a substrate provided in a microfluidic chip according to another embodiment of the present invention.
[Fig. 10] Fig. 10 is a figure showing a cross-section of a detection channel of a microfluidic chip.
[Fig. 11] Fig. 11 is a schematic diagram for explaining another embodiment of a PCR device for performing a thermal cycle and PCR in which a microfluidic chip can be used.

### DESCRIPTION OF EMBODIMENTS

The embodiments of the present invention are now described in the following. The same or equivalent constituting elements, members, and processes illustrated in each drawing shall be denoted by the same reference numerals, and duplicative explanations will be omitted appropriately. Further, the embodiments do not limit the invention and are shown for illustrative purposes, and not all the features described in the embodiments and combinations thereof are necessarily essential to the invention.

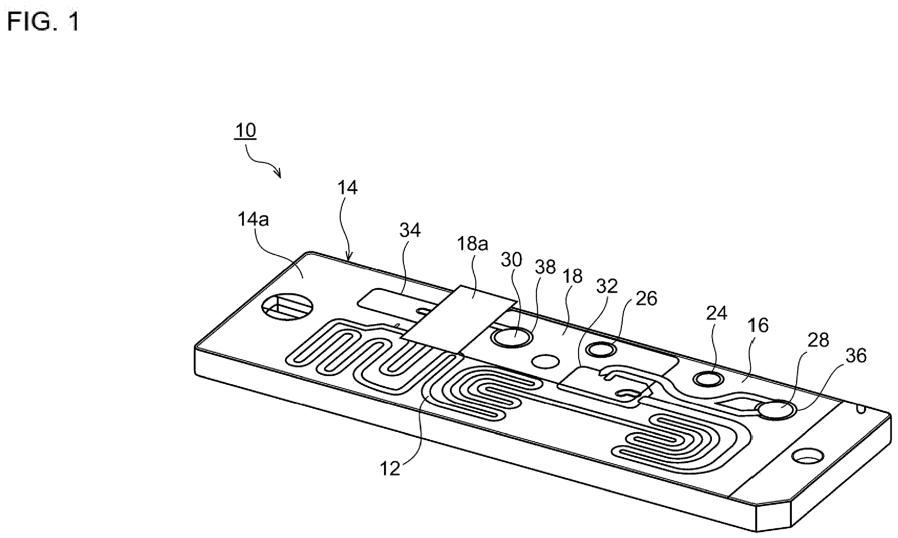

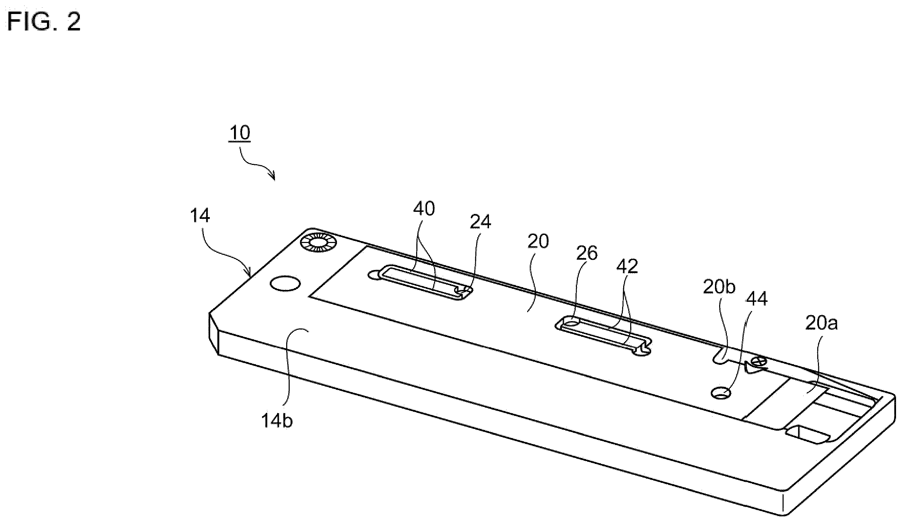

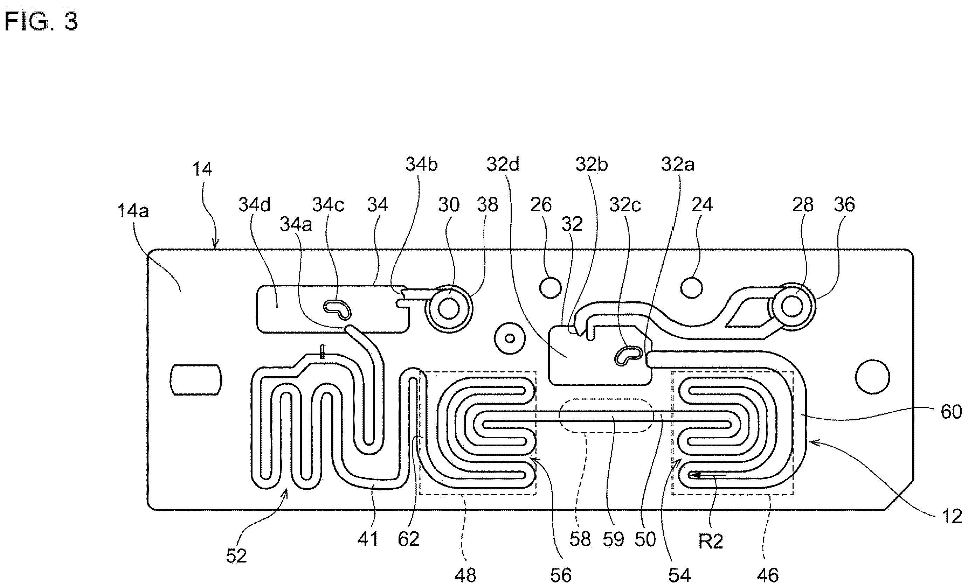

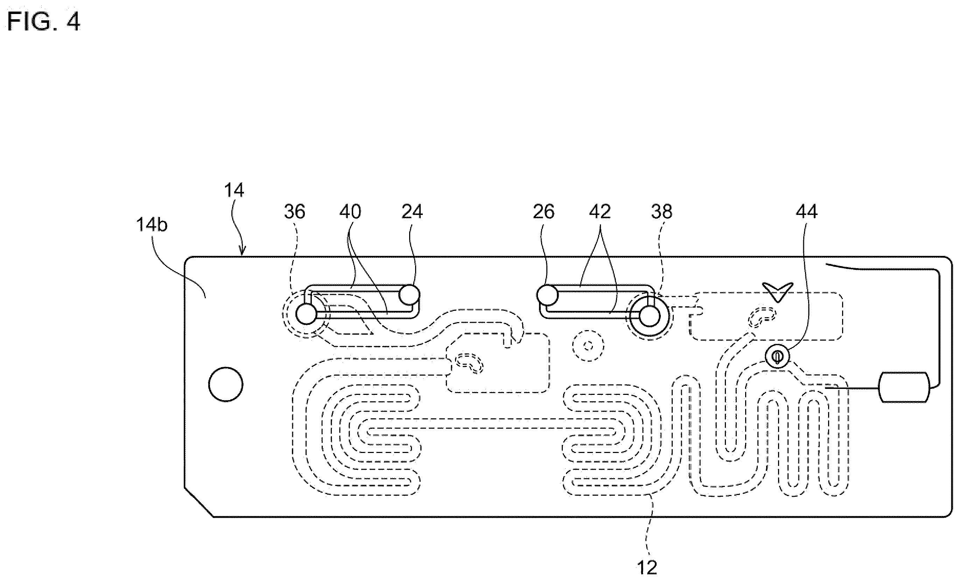

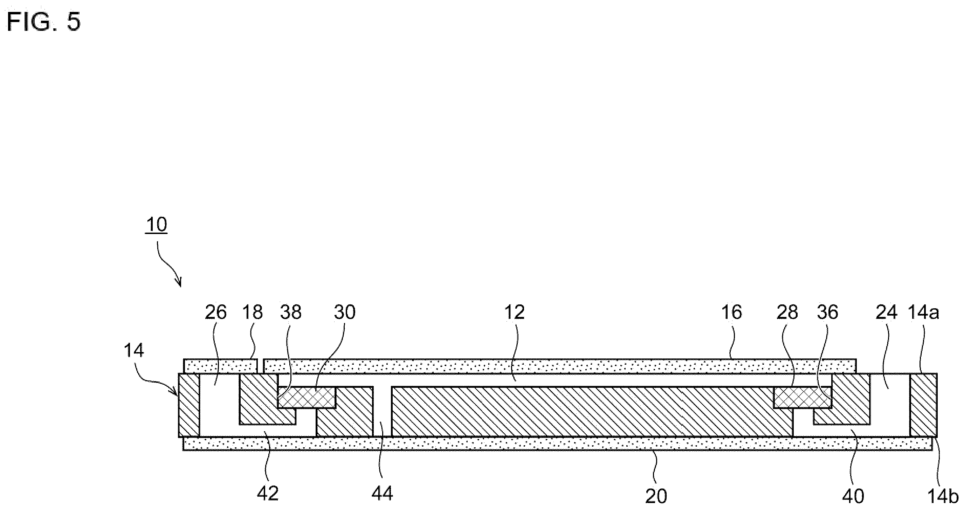

Fig. 1 is a perspective view showing the first surface side of a microfluidic chip 10 according to an embodiment of the present invention. Fig. 2 is a perspective view showing the second surface side of the microfluidic chip 10. Fig. 3 is a plan view of a first surface 14a of a substrate 14 provided in the microfluidic chip 10. Fig. 4 is a plan view of a second surface 14b of the substrate 14 provided in the microfluidic chip 10. Fig. 5 is a conceptual diagram for explaining a cross-sectional structure of the microfluidic chip 10. Fig. 5 is a diagram for explaining the positional relationship between a channel, a film, a filter, and a substrate, and it should be noted that the diagram is different from a cross-sectional view of the implemented microfluidic chip.

The microfluidic chip (also referred to as a "fluid chip," a "(micro) channel chip," a "reaction processing chip," a "reaction processing vessel," a "nucleic acid amplification chip," a "DNA chip" or a "measurement chip") 10 includes a channel 12 in which a sample that is subjected to a thermal cycle or reaction moves. The microfluidic chip 10 includes a resin substrate 14 in which a groove-shaped channel 12 is mainly formed where a sample moves to a first surface 14a, a first sealing film 16 and a second sealing film 18 attached to the first surface 14a of the substrate 14a, and a third sealing film 20 attached to a second surface 14b of the substrate. Further, in the microfluidic chip 10, a first filter 28 and a second filter 30 may be provided near both ends of the channel 12 of the substrate 14 for the purpose of suppressing contamination to the channel 12.

As shown in Fig. 3, the first surface 14a of the substrate 14 of the microfluidic chip 10 is provided with at least a groove-shaped channel 12, air coupling ports 24 and 26 that are arranged at the respective ends of the channel 12 and are for attaching the output of a pump that connects to the channel 12 and pressurizes or feeds air into the channel, room-shaped chambers 32 and 34 provided on the channel 12; and filter spaces 36 and 38 for placing the filters 28 and 30 for preventing contamination of the channel 12. In the groove-shaped channel 12, samples to be subjected to PCR or thermal cycles are intended to be moved.

As shown in Fig. 4, the second surface 14b of the substrate 14 is provided with groove-shaped air ducts 40 and 42 for connecting between the air coupling ports 24 and 26 and the filter spaces 36 and 38, and a sample introduction port (sample introduction hole) 44 for introducing a sample into the channel 12. The groove-shaped air ducts 40 and 42 may be intended as channels through which air mainly passes for air feeding or pressurization from pumps or the like connected to the air coupling ports 24 and 26. Providing the channel 12, the air ducts 40 and 42, and the like on the two opposing principal surfaces (the first surface 14a and the second surface 14b) of the substrate 14 in this manner helps to make effective use of the principal surfaces of the substrate 14.

The channel 12 and the air coupling ports 24 and 26 may be provided on opposing surfaces, and the channel 12 and the filter spaces 36 and 38 may be provided on the same surface. Further, the first surface 14a and the second surface 14b described above may include elements other than those mentioned above.

The substrate 14 of the microfluidic chip 10 is preferably formed of a material that is stable under temperature changes and is resistant to a sample solution that is used. Further, the substrate 14 is preferably formed of a material that has good moldability, a good barrier property, and a low self-fluorescent property. As such a material, a resin such as acryl, polypropylene, silicone, or the like, particularly a cyclic polyolefin resin is preferred. When the substrate is made of resin, production in large quantities and with high quality is possible by injection molding using a mold.

The size of the substrate 14 of the microfluidic chip 10 is not particularly limited, and the size does not matter as long as the chemical and physical action in the channel formed in the substrate are satisfied. On the other hand, if there is a request for reducing the size of the device on which the microfluidic chip is mounted, the microfluidic chip and the substrate constituting the microfluidic chip can also be made smaller or thinner.

Further, the substrate 14 of the microfluidic chip 10 may be in the form of a parallel plate with good formability, portability, and handling. Alternatively, the substrate 14 may be partially uneven due to, e.g., an increase in the thickness of the portion having a relatively high channel density, a decrease in the thickness of the portion of the substrate corresponding to a fluorescence detection region described below, and an increase in the thickness of the portion of the substrate corresponding to the area where a filter described below is installed.

The size of the substrate 14 of the microfluidic chip 10 is, for example, a thickness of 1 mm to 10 mm, preferably 2 mm to 6 mm, and a rectangular shape of W × L in planar view where W is 10 mm to 60 mm, preferably 20 mm to 35 mm, and H is 45 mm to 100 mm, preferably 60 mm to 85 mm.

Further, the microfluidic chip 10 may be made of a transparent resin. The microfluidic chip 10 is intended to be used for amplification of nucleic acids contained in a sample by a real-time PCR method. When the reaction of a sample containing nucleic acid progresses within the channel of the microfluidic chip 10, the progress and completion of the reaction can be known by detecting and monitoring the fluorescence emitted from the sample in real time. As a part to be subjected to optical measurement such as detection of fluorescence from a sample in the channel or irradiation of excitation light that induces fluorescence, the microfluidic chip 10 is preferably made of a transparent material.

Further, as long as the position at which the irradiation of excitation light or the detection of fluorescence has an optical effect is fixed, only that site may be made transparent, and the rest of the portion may be made of, for example, black resin. The fixed fluorescence detection position can be used as a fluorescence detection region (detection region), and the portion of the substrate 14 corresponding to that site can be made transparent, while the other portion can be made black. In this case, by making the portions that give and receive optical effects on a sample transparent, it is possible to exchange such effects, while, for example, by making the portions of the substrate 14 out of black resin that correspond to the respective temperature regions maintained at the required temperatures for applying a thermal cycle to the sample, it is possible to expect improved thermal conductivity and the like, resulting in a more efficient heating effect. Such a production method allows for convenient production by joining different types of materials in a mold using a so-called multicolor molding method.

The groove-like channel 12 is formed on the first surface 14a of the substrate 14. In the microfluidic chip 10, most of the channel 12 is formed in a groove shape exposed on the first surface 14a of the substrate. Further, the groove-shaped air ducts 40 and 42 are formed in a groove shape exposed on the second surface 14b of the substrate 14. By achieving such a form, the substrate 14 provided on two principal surfaces facing the channel 12 and the like is easily molded by injection molding using a mold. In order to seal a groove formed on the substrate 14 so as to make use of the groove as a channel 12, a channel sealing film is attached on each surface of the substrate 14. The dimensions of the channel 12 are, for example, about 0.2 mm to 1.5 mm in width and 0.2 mm to 2 mm in depth, and the dimensions of the channel 12 may be 0.7 mm in width and 0.7 mm in depth. Further, in order to produce the substrate 14 more advantageously in an industrial manner by the injection molding method, the structure of the channel 12 may include a side surface having a certain angle with respect to the first surface 14a and second surface 14b of the substrate 14, which is referred to as a so-called "draft angle". The angle of the taper may be 1° to 30° and may be 3° to 15°.

The sealing films 16, 18, and 20 for sealing the channel 12, the sample introduction port 44, the air coupling ports 24 and 26, the chambers 32 and 34, and the filters 28 and 30 may be sticky on one of the principal surfaces thereof or may have a functional layer that exhibits stickiness or adhesiveness through pressing, energy irradiation with ultraviolet rays or the like, heating, etc., formed on one of the principal surfaces. Thus, the sealing films 16, 18, and 20 have a function of being easily able to become integral with the first and second surfaces 14a and 14b of the substrate 14 while being in close contact with the first and second surfaces 14a and 14b. The sealing films 16, 18, and 29 are desirably formed of a material having low or preferably almost zero autofluorescence, including an adhesive. In this respect, a transparent film made of resin such as a cycloolefin polymer, polyester, polypropylene, polyethylene or acrylic is suitable but is not limited thereto. Further, the sealing films 16, 18, and 20 may be formed of plate-shaped glass or resin. Since rigidity can be expected in this case, the sealing films are useful for preventing warpage and deformation of the microfluidic chip 10.

A first filter 28 is provided near one end of the channel 12. A second filter 30 is provided near the other end of the channel 12. A pair of the first and second filters 28 and 30 provided at the respective ends of the channel 12 is intended to prevent contamination in the channel or to a sample so as not to interrupt the amplification of target DNA and the detection of the DNA by PCR or so as not to cause the quality of the target DNA to deteriorate. Regarding the dimensions of the first filter 28 and the second filter 30, the first filter 28 and the second filter 30 are formed so as to fit without any gap in the filter spaces 36 and 38 formed on the first surface 14a of the substrate 14. The first filter 28 and the second filter 30 may be, for example, substantially cylindrical or substantially disk-shaped, and the size thereof may be 2 mm to 10 mm in diameter ϕ of the bottom surface, preferably 2.5 mm to 7.5 mm, and more preferably 3 mm to 5 mm. The first filter 28 and the second filter 30 may be 1 mm to 2.8 mm in height. The height of the first filter 28 and the second filter may be smaller than the depth of the filter spaces 36, 38, and may be a height such that the first filter 28 and the second filter 30 do not protrude from the surface of the substrate 14 when stored in the filter spaces 36, 38, respectively.

A first air coupling port 24 connecting to one end of the channel 12 via a first air duct 40 and the first filter 28 is formed in the substrate 14. In the same manner, a first air coupling port 26 connecting to the other end of the channel 12 via a second air duct 42 and the second filter 30 is formed in the substrate 14. The pair of air coupling ports, the first air coupling port 24 and the second air coupling port 26, is formed so as to be exposed on the first surface 14a of the substrate 14.

In the present embodiment, those with good low impurity characteristics and with air permeability and water repellency or oil repellency are used as the first filter 28 and the second filter 30. The first filter 28 and the second filter 30 are preferably, for example, porous resins, sintered compacts of resin, or the like, and examples of a fluorine-containing resin include, although not limited to, PTFE (polytetrafluoroethylene), PFA (perfluoroalkoxyalkane), FEP (perfluoroethylene propene copolymer), ETFE (ethylene tetrafluoroethylene copolymer), etc. Further, as a filter made of PTFE (polytetrafluoroethylene), although not limited to this, PF020 (manufactured by ADVANTEC Group) or the like can be used. Further, those whose surface is water-repellent treated through coating with a fluorine-containing resin can be used as the first filter 28 and the second filter 30. Other filter materials include polyethylene, polyamide, polypropylene, and the like, and any material that can prevent contamination of the sample to be subjected to PCR and that does not interfere with PCR may be used. A material that has a property of allowing the passage of the air while preventing the passage of a liquid is even better, and the performance and the quality of the material are not limited as long as the material satisfies some of these requirements for the required performance. Further, the pore diameter of the first filter 28 and the second filter 30 may be 0.5 µm to 20 µm, preferably 2.5 µm to 10 µm.

A part of the channel 12 includes a thermal cycle region where the control of multiple levels of temperature is possible by a PCR device described later between a pair of filters: the first filter 28 and the second filter 30. By setting the microfluidic chip 10 in the PCR device having a temperature control system including a heater or the like, a part of the channel 12 of the microfluidic chip 10 is heated by the temperature control system and maintained at a desired temperature. The temperature is controlled so as to be maintained at multiple temperatures necessary to apply a thermal cycle and produce a reaction such as PCR. The reaction includes a reaction in which the nucleic acid contained in the sample is thermally denatured, an annealing reaction, and an elongation reaction. The temperature controlled at a part of the channel 12 may be a temperature that causes these reactions. A thermal cycle can be applied to a sample by moving the sample such that the sample continuously moves in a reciprocating motion in the reaction region where the multiple levels of temperature are maintained as described.

In the present embodiment, the thermal cycle region includes a first temperature region 46 and a second temperature region 48. When the microfluidic chip 10 is mounted on the PCR device described later, the first temperature region 46 is maintained at a relatively high temperature (for example, about 95°C), and the second temperature region 48 is maintained to be lower than the first temperature region 46 (for example, about 62°C). Nucleic acid and the like contained in the sample may undergo a thermal denaturation reaction in the channel included in the first temperature region 46, and may undergo an annealing reaction and an elongation reaction in the channel included in the second temperature region 48. The first temperature region 46 and the second temperature region 48 may be referred to as a "high temperature region" and a "medium temperature region," respectively, from the viewpoint of their respective high or low temperatures. Further, the first temperature region 46 and the second temperature region 48 may be referred to as a "denaturation region" and an "annealing and elongation region" from the viewpoint of the attributes of the reaction.

One end of the first temperature region 46 connects to the first chamber 32. Further, the first chamber 32 connects to the first air coupling port 24 via the first filter 28 and the first air duct 40.

The other end of the first temperature region 46 connects to the second temperature region 48 via a connection channel 50. One end of the second temperature region 48 connects to the first temperature region 46 via the connection channel 50, and the other end connects to a third meandering channel region 52. When the first temperature region 46 and the second temperature region 48 including the meandering channels 54 and 56 composed of a straight channel and a curved channel are considered as the first and second meandering channel regions, respectively, a region including meandering channels other than those belonging to the first temperature region 46 and the second temperature region 48 will be explained as the third meandering channel region. One end of the channel belonging to the third meandering channel region 52 connects to the second temperature region 48 through the connection channel 41, and the other end connects to the second chamber 34. Further, the second chamber 34 connects to the second air coupling port 26 via the second filter 30 and the second air duct 42. In another embodiment, a second chamber may not be provided. In terms of the role and function of the chambers, the second chamber 34 is less important than the first chamber 32 in terms of provision and can therefore be omitted, in which case the channels belonging to the third meandering channel region 52 connects directly to the second filter 30.

In the present embodiment, the channels belonging to the third meandering channel region 52 are channels not directly related to the thermal cycle or PCR, and include meandering channels including a straight channel and a curved channel. The channel may be used as a buffer-like channel for setting a sample to be subjected to PCR on stand-by by having a structure in which the sample introduction port 44 for introducing a sample into a channel is connected to the channel within the third meandering channel region 52. The third meandering channel region 52 may have the role of a region including a channel for temporary stand-by for a sample when the sample is introduced from the sample introduction port, and may have a "sample stand-by region" function.

The regions intended to function as the first temperature region 46 and the second temperature region 48, respectively, include meandering channels 54 and 56 where a turn is continuously made by combining curved channels and straight channels. It can be considered that a plurality of curved channels is connected to straight channels. When such a meandering channel is used, a limited effective area such as a heater constituting a temperature control system described later can be effectively used. This contributes to reducing the temperature variation within each temperature region, and has the advantage that the actual size of the microfluidic chip 10 can be reduced, thereby contributing to the miniaturization of the PCR device. On the other hand, the first temperature region 46 and the second temperature region 48 are connected by the connection channel 50. In the connection channel 50, when the microfluidic chip 10 is mounted in the PCR device described later, a region (referred to as a "fluorescence detection region" or simply a "detection region") 58 may be provided that is irradiated with excitation light in order to detect fluorescence from a sample flowing inside the channel. The channel included in this fluorescence detection region 58 is referred to as a detection channel 59.

The fluorescence detection region may be provided in a region other than the connection channel 50. For example, a part of a channel that belongs to the first temperature region 46 ("high temperature region" or "denaturing region"), the second temperature region 48 ("medium temperature region" or "annealing and elongation region"), or the third meandering channel region 52 (sample stand-by region) may be formed as a fluorescence detection region to detect fluorescence from a sample in the channel. Further, the movement of a sample solution may be detected using a part of the channel that belongs to the connection channel 50, the first temperature region 46 ("high temperature region" or "denaturing region"), the second temperature region 48 ("medium temperature region" or "annealing and elongation region"), or the third meandering channel region 52 (sample stand-by region) as a detection region by an optical detector other than a fluorescent detector capable of detecting the movement of the sample solution. Further, fluorescence may be detected from a sample flowing inside the connection channel 50 by a fluorescent detector and the movement of a sample solution may be detected using a part of the channel that belongs to the first temperature region 46 ("high temperature region" or "denaturing region"), the second temperature region 48 ("medium temperature region" or "annealing and elongation region"), or the third meandering channel region 52 (sample stand-by region) as a detection region by an optical detector other than a fluorescent detector. For fluorescence detection performed in the connection channel 50, the first temperature region 46 ("high temperature region" or "denaturing region"), the second temperature region 48 ("medium temperature region" or "annealing and elongation region"), or the third meandering channel region 52 (sample stand-by region) or optical detection other than detection of fluorescence, light of different wavelengths may be detected.

Further, fluorescence may be detected from a sample flowing inside the connection channel 50 by a fluorescent detector and the movement of a sample solution may be detected using a part of the channel that belongs to the first temperature region 46 ("high temperature region" or "denaturing region"), the second temperature region 48 ("medium temperature region" or "annealing and elongation region"), or the third meandering channel region 52 (sample stand-by region) as a detection region by an optical detector other than a fluorescent detector.

For fluorescence detection performed in the connection channel 50, the first temperature region 46 ("high temperature region" or "denaturing region"), the second temperature region 48 ("medium temperature region" or "annealing and elongation region"), or the third meandering channel region 52 (sample stand-by region) or optical detection other than detection of fluorescence, light of different wavelengths may be detected. For a light source (LED) irradiating a channel in the denaturing temperature region, for example, those with an excitation wavelength of 525 nm may be used. For a light source (LED) irradiating the connection channel, for example, those with an excitation wavelength of 470 nm may be used. For a light source (LED) irradiating a channel in the elongation and annealing temperature region, for example, those with an excitation wavelength of 630 nm may be used.

In addition to aspects including a straight channel, the connection channel 50 may include a curved channel, may include a gently curved channel, may be a channel including a straight channel and a curved channel, and may be a meandering channel.

Further, the connection channel 50 corresponds to a portion in which a part of a fluorescence detection device for detecting the fluorescence of a sample is placed. Therefore, the interval between the first temperature region 46 and the second temperature region 48 may be relatively long, for example, the interval between the first temperature region 46 and the second temperature region 48 may be longer than a straight channel included in the first temperature region 46 and/or the second temperature region 48. Since a part of the fluorescence detection device arranged in the connection channel 50 has a predetermined volume, it is advantageous to set the interval to have a certain distance or more in order to arrange some parts of the fluorescence detection device. Further, there is an advantage that the longer the interval between the first temperature region 46 and the second temperature region 48 becomes, the smaller the thermal interference between the two temperature regions becomes. When the thermal interference between the temperature regions is small, the heat and the like provided from the device through a temperature control means such as a heater can be effectively used, contributing to power saving of the device.

On the other hand, since there is an advantage of reducing the size of the PCR device and the microfluidic chip 10 (portability, weight, etc.), the interval between the first temperature region 46 and the second temperature region 48 may be, for example, shorter than half the length L of the long side of the microfluidic chip 10 in a plan view. In this case, the degree of freedom in designing the microfluidic chip, such as mounting other elements and arranging margins and blank spaces, can be improved, making it easier to ensure the size and portability required for a PCR device.

The first chamber 32 is arranged between the thermal cycle region and the first air coupling port 24. More specifically, the first chamber 32 is arranged between the first temperature region 46 of the thermal cycle region and the first filter 28. The sample in the channel moves toward the first temperature region 46 by, for example, pressurization or air feeding from the second air coupling port 26. The first chamber 32 has the role of preventing a sample from being pushed too much or from overrunning the first temperature region 46 and reaching the first filter 28.

If the first chamber 32 is not provided, a sample may overrun the first temperature region 46 and reach the first filter 28 when the sample is pushed for an excessively long time or with an excessively large pressure. When at least a part of the sample reaches the filter, wetting of the filter by the sample occurs since the sample is in a liquid state. In such a case, a certain amount of a part of the sample that is not subjected to the thermal cycle remains, and erroneous measurement may occur in the progress of PCR or the like according to the concentration of amplified DNA or the like. Further, a part of the sample that has wetted the filter may evaporate and enter the liquid delivery system of the PCR device, which may result in, for example, a decrease in the reaction of the next lot or a decrease in the ability to detect nucleic acids.

In the microfluidic chip 10 according to the present embodiment, by providing a first chamber 32 having a certain volume in the channel, the sample can remain within the first chamber 32 and be stopped even when the sample passes a predetermined intended stop position in the thermal cycle region, thereby preventing the flow of the sample toward the first air coupling port 24. As in the microfluidic chip 10 according to the present embodiment, when the first filter 28 for preventing contamination to the channel 12 is provided between the first air coupling port 24 and the thermal cycle region, the sample can be stored in the first chamber 32. Thus, the flow of the sample toward the first filter 28 can be suppressed, and the sample can be prevented from reaching the first filter 28.

The first chamber 32 may be formed in a concave shape being exposed on the first surface 14a of the substrate 14 in the same manner as in other channel forms, and the first chamber 32 is sealed by attaching a sealing film to the first surface 14a. Since the first chamber 32 is provided for the purpose of preventing or suppressing the flow of the sample toward the first filter 28, even if substantially all of the sample enters the chamber, the progression to the first filter may be suppressed there.

Given that the volume of the sample that is subjected to the thermal cycle or PCR is V₀ [mm³], the volume V₁ [mm³] of the first chamber 32 is preferably expressed as 1.0 × V₀ ≤ V₁ ≤ 6.0 × V₀, and more preferably 2.0 × V₀ ≤ V₁ ≤ 5.0 × V₀. Further, the volume V₁ [mm³] of the first chamber may be 25 mm³ to 100 mm³ or 40 mm³ to 80 mm³.

If the volume of the first chamber 32 is excessively large, pressure loss will occur, which is disadvantageous in terms of pressurization and air feeding from the air coupling ports. On the other hand, if the volume of the first chamber 32 is excessively small, all of the sample that has passed through the thermal cycle region cannot be absorbed, and a part of the sample will flow toward the first filter 28 and the first air coupling port 24. Thus, this is not effective. The volume of the first chamber 32 required may obviously depend on the volume of the sample to be subjected to PCR or thermal cycle. Further, when the volume of the sample to be subjected to PCR or the like is determined in advance, the volume of the first chamber 32 may be specified.

When the first chamber 32 is substantially rectangular in plan view, the size thereof may be 5 mm to 15 mm on the long side, 3 mm to 15 mm on the short side, and 0.25 mm to 3 mm in depth. Further, the upper limit of the depth may be less than the thickness of the substrate. When the first chamber 32 is substantially circular (including a circular shape, an elliptical shape, an oval shape, and the like) in plan view, the size thereof may be 5 mm to 15 mm on the major axis, 3 mm to 15 mm on the minor axis, and 0.25 mm to 3 mm in depth. Further, the upper limit of the depth may be less than the thickness of the substrate. If the first chamber 32 is substantially rectangular, the bottom surface thereof may be 5 mm to 15 mm on the long side and may be 3 mm to 15 mm on the short side, and if the first chamber 32 is substantially circular, the bottom surface thereof may be 5 mm to 15 mm on the major axis and may be 3 mm to 15 mm on the minor axis. Further, in the first chamber 32, the bottom surface 32d thereof may be provided parallel to the first surface 14a of the substrate 14.

Further, as shown in Fig. 3, the first chamber 32 includes a first connection 32a, which is a connection (or opening) with a channel end of the channel extending from the first temperature region 46 of the thermal cycle region, and a second connection 32b, which is a connection to a channel end of the channel heading toward the first filter 28. The first chamber 32 has a first structure 32c provided to divide a virtual straight line connecting the first connection 32a and the second connection 32b. The first structure 32c prevents a liquid sample that has entered the first chamber 32 from the first connection 32a from heading directly to the second connection 32b and contributes to preventing the entry of the sample in the direction of the first filter 28. The first structure 32c may have any shape or arrangement as long as a part of the first structure 32c divides the virtual straight line.

The first structure 32c may be provided near the first connection 32a. This is because the purpose is to control the flow of sample entering from the first connection 32a.

The width of the first connection 32a may be smaller than the length of the first structure 32c in the direction specifying the width of the first connection 32a. In this case, it can be expected that the flow of the sample that has entered from the first connection 32a is further suppressed. When the width of the first connection 32a is denoted as W₁ and the length of the first structure 32c parallel to the direction of W₁ is denoted as Wₛₜᵣ₋₁, the following may be established: W₁ < _{Wstr-1} or W₁ < Wₛₜᵣ₋₁ < 3 × W₁.

Further, the first structure 32c may be provided so as to bend the flow of a sample in a certain direction. In this case, it can be expected that the liquid sample that has entered from the first connection 32a is further prevented from heading directly toward the second connection 32b. The first structure 32c may be provided in a shape convex from the bottom surface 32d of the first chamber 32. When the first chamber 32 is provided in a concave shape with respect to the substrate 14, providing the first structure 32c convexly from the bottom surface 32d facilitates the formation of the first structure 32c by injection molding.

In Fig. 3, the first structure 32c is provided convexly from the bottom surface 32d of the first chamber 32 toward the first surface 14a of the substrate 14, and a partially bent oval shape is shown as an example of the plan-view shape of the first structure 32c. This shape shown as an example can also be said to be two oval shapes overlapping each other such that the central axes of the shapes intersect. It is suggested that this shape has a function of bending the flow when a sample enters the first chamber 32 from the first connection 32a in such a manner that the flow from the channel before the first connection 32a becomes straight. The plan-view shape of the first structure 32c of such a function may be, in addition to a curved oval shown as an example, a crescent shape having one curved side, or a shape in which a part of a concave portion faces the first connection 32a.

Since the first chamber 32 is not actively intended for liquid sample entry, there may be a gap (difference in height) between the bottom surface of the second connection 32b and the bottom surface 32d of the first chamber 32. In this case, since a sample that has entered the first chamber 32 is less likely to escape from first chamber 32 by going over the gap, the sample is further prevented from reaching the first filter 28. Given that the gap between the bottom surface of the first chamber 32 and the bottom surface of the second connection 32b is denoted as G₁₋₂, the following is preferably established: 0.05 mm ≤ G₁₋₂ [mm] ≤ 1 mm. The first chamber 32 may be a single chamber or may include two or more chambers where the chambers are connected by channels.

The first chamber may have functions other than preventing sample overrun. For example, the first chamber may be used as a reaction chamber. More specifically, a reagent may be held in the chamber in advance, and PCR may be performed after the reagent reacts with a sample in the chamber. A freeze-dried reagent can be shown as an example of the reagent held in advance in the chamber. If the first chamber contains two or more chambers, one chamber may be used as an overrun prevention chamber and the other chamber may be used as a reaction chamber. In this case, by using one chamber closer to the filter as the overrun prevention chamber and one chamber farther from the filter as the reaction chamber, the overrun sample from the reaction chamber can be further prevented from reaching the filter. The reaction chamber may be heated, cooled, or maintained at a temperature with a temperature control system. The reaction can be controlled by heating, cooling or maintaining the temperature.

The second chamber 34 is arranged between the thermal cycle region and the second air coupling port 26. More specifically, the second chamber 34 is arranged between the third meandering channel region 52 and the second filter 30. A sample in the channel moves toward the second temperature region 48 by, for example, pressurization or air feeding from the first air coupling port 26. The second chamber 34 has the role of preventing a sample from being pushed too much or from overrunning the second temperature region 48 or the third meandering channel region 52 and reaching the second filter 30. If the second chamber 34 is not provided, a sample may overrun the second temperature region 48 or the third meandering channel region 52 and reach the second filter 30 when the sample is pushed for an excessively long time or with an excessively large pressure. When at least a part of the sample reaches the filter, wetting of the filter by the sample occurs since the sample is in a liquid state. In such a case, a certain amount of a part of the sample that is not subjected to the thermal cycle remains, and erroneous measurement may occur in the concentration of amplified DNA or the like. Further, a part of the sample that has wetted the filter may evaporate and enter the liquid delivery system of the PCR device, which may result in, for example, a decrease in the reaction of the next lot or a decrease in the ability to detect nucleic acids.

On the other hand, since the third meandering channel region 52 is provided between the second temperature region 48 and the second filter 30, it is intuitively understood that a sample overrunning the second temperature region 48 has a smaller probability of reaching the filter compared to the circumstances on the first temperature region side. Therefore, the second chamber 34 has no higher priority than the first chamber 32 and may be omitted in some cases.

The second chamber 34 may be formed in a concave shape being exposed on the first surface 14a of the substrate 14 in the same manner as in other channel forms, and the second chamber 34 is sealed by attaching a sealing film to the first surface 14a. Since the second chamber 34 is provided for the purpose of preventing or suppressing the flow of the sample toward the second filter 30, even if substantially all of the sample enters the chamber, the progression to the second filter may be suppressed there.

Given that the volume of the sample that is subjected to the thermal cycle or PCR is V₀ [mm³], the volume V₂ [mm³] of the second chamber 34 is preferably expressed as 1.0 × V₀ ≤ V₂ ≤ 6.0 × V₀, and more preferably 2.0 × V₀ ≤ V₂ ≤ 5.0 × V₀. Further, the volume V₂ [mm³] of the second chamber 34 may be 25 mm³ to 100 mm³ or 40 mm³ to 80 mm³.

If the volume of the second chamber 34 is excessively large, pressure loss will occur, which is disadvantageous in terms of pressurization and air feeding from the air coupling ports. On the other hand, if the volume of the second chamber 34 is excessively small, all of the sample that has passed through the thermal cycle region cannot be absorbed, and a part of the sample will flow toward the second filter 30 and the second air coupling port 26. Thus, this is not effective. The volume of the second chamber 34 required may obviously depend on the volume of the sample to be subjected to PCR or thermal cycle. Further, when the volume of the sample to be subjected to PCR or the like is determined in advance, the volume of the second chamber 34 may be specified.

When the second chamber 34 is substantially rectangular in plan view, the size thereof may be 5 mm to 15 mm on the long side, 3 mm to 15 mm on the short side, and 0.25 mm to 3 mm in depth. Further, the upper limit of the depth may be less than the thickness of the substrate. When the second chamber 34 is substantially circular (including a circular shape, an elliptical shape, an oval shape, and the like) in plan view, the size thereof may be 5 mm to 15 mm on the major axis, 3 mm to 15 mm on the minor axis, and 0.25 mm to 3 mm in depth. Further, the upper limit of the depth may be less than the thickness of the substrate. If the second chamber 34 is substantially rectangular, the bottom surface thereof may be 5 mm to 15 mm on the long side and may be 3 mm to 15 mm on the short side, and if the second chamber 34 is substantially circular, the bottom surface thereof may be 5 mm to 15 mm on the major axis and may be 3 mm to 15 mm on the minor axis. Further, in the second chamber 34, the bottom surface 32d thereof may be provided parallel to the first surface 14a of the substrate 14.

Further, as shown in Fig. 3, the second chamber 34 includes a third connection 34a, which is a connection to a channel end of the channel extending from the third meandering channel region 52, and a fourth connection 34b, which is a connection to a channel end of the channel heading toward the second filter 30. In the second chamber 34, a second structure 34c may be provided near the third connection 34a in order to control the flow of a sample entering from the third connection 34a.

The width of the third connection 34a may be smaller than the length of the second structure 34c in the direction specifying the width of the third connection 34a. In this case, it can be expected that the flow of the sample that has entered from the third connection 34a is further suppressed. When the width of the third connection 34a is denoted as W₃ and the length of the second structure parallel to the direction of W₃ is denoted as Wₛₜᵣ₋₂, the following may be established: W₃ < _{Wstr-2} or W₃ < Wₛₜᵣ₋₂ < 3 × W₃.

Further, the second structure 34c may be provided so as to bend the flow of a sample in a certain direction. In this case, it can be expected that the liquid sample that has entered from the third connection 34a is further prevented from heading directly toward the fourth connection 34b. The second structure 34c may be provided in a shape convex from the bottom surface 34d of the second chamber 34. When the second chamber 34 is provided in a concave shape with respect to the substrate 14, providing the second structure 34c convexly from the bottom surface 34d facilitates the formation of the first structure 32c by injection molding.

In Fig. 3, the second structure 34c is provided convexly from the bottom surface 34d of the second chamber 34 toward the first surface 14a of the substrate 14, and a partially bent oval shape is shown as an example of the plan-view shape of the second structure 34c. This shape shown as an example can also be said to be two oval shapes overlapping each other such that the central axes of the shapes intersect. It is suggested that this shape has a function of bending the flow when a sample enters the second chamber 34 from the third connection 34a in such a manner that the flow from the channel before the third connection 34a becomes straight. The plan-view shape of the second structure 34c of such a function may be, in addition to a curved oval shown as an example, a crescent shape having one curved side, or a shape in which a part of a concave portion faces the third connection.

Since the second chamber 34 is not actively intended for liquid sample entry, there may be a gap between the bottom surface of the fourth connection 34b and the bottom surface 34d of the second chamber 34. In this case, since a sample that has entered the second chamber 34 is less likely to escape from second chamber 34 by going over the gap, the sample is further prevented from reaching the second filter 30. Given that the gap between the bottom surface 34d of the second chamber 34 and the bottom surface of the fourth connection 34b is denoted as G₂₋₄, the following is preferably established: 0.05 mm ≤ G₂₋₄ [mm] ≤ 1 mm. The second chamber 34 may be a single chamber or may include two or more chambers where the chambers are connected by channels.

The second chamber may have functions other than preventing sample overrun. For example, the first chamber may be used as a reaction chamber. More specifically, a reagent may be held in the chamber in advance, and PCR may be performed after the reagent reacts with a sample in the chamber. A freeze-dried reagent can be shown as an example of the reagent held in advance in the chamber. If the second chamber contains two or more chambers, one chamber may be used as an overrun prevention chamber and the other chamber may be used as a reaction chamber. In this case, by using one chamber closer to the filter as the overrun prevention chamber and one chamber farther from the filter as the reaction chamber, the overrun sample from the reaction chamber can be further prevented from reaching the filter. The reaction chamber may be heated, cooled, or maintained at a temperature with a temperature control system. The reaction can be controlled by heating, cooling or maintaining the temperature.

The microfluidic chip may include a third or fourth chamber. The third or fourth chamber may be used for functions other than preventing sample overrun, for example, as a reaction chamber. More specifically, a reagent may be held in the chamber in advance, and PCR may be performed after the reagent reacts with a sample in the chamber. A freeze-dried reagent can be shown as an example of the reagent held in advance in the chamber. When the third or fourth chamber is used as a reaction chamber, the chamber may be heated, cooled, or maintained at a temperature with a temperature control system. For example, the chamber may be provided in the thermal cycle region (including the first and second temperature regions). The reaction can be controlled by heating, cooling or maintaining the temperature.

As mentioned above, the first temperature region 46 and the second temperature region 48 each include a meandering channel where turns are continuously made by combining curved channels and straight channels. That is, the first temperature region 46 includes a first meandering channel 54, and the second temperature region 48 includes a second meandering channel 56. Since such a meandering channel can effectively utilize the limited area of the substrate, the substantial size of the microfluidic chip 10 can be reduced, contributing to the downsizing of the PCR device. Further, a limited effective area of a heater constituting a temperature control system described later can be effectively used, and temperature variance in the thermal cycle region is easily reduced.

A first widened channel 60 may be provided between the first meandering channel 54 and the first chamber 32. The first widened channel 60 is provided adjacent to the channel belonging to the first temperature region 46 and on the back side of the meandering channel 54 when viewed from the connection channel 50. A second widened channel 62 may be provided between the second meandering channel 56 and the channel belonging to the third meandering channel region 52. The second widened channel 62 is formed adjacent to the channel belonging to the second temperature region 48 and on the back side of the channel belonging to the second temperature region 48 when viewed from the connection channel 50.

The first widened channel 60 is provided such that the cross-sectional area thereof is larger than the cross-sectional area of the first meandering channel 54. In the same way, the second widened channel 62 is provided such that the cross-sectional area thereof is larger than the cross-sectional area of the second meandering channel 56. As will be described later in detail, the first widened channel 60 and the second widened channel 62 each have a role of exerting a braking action on the sample flowing through the first meandering channel 54 and the second meandering channel 56, respectively. This utilizes Bernoulli's law, which states that the speed of fluid passing through a flow tube is inversely proportional to the cross-sectional area of the flow tube, and it is not uncommon to create a configuration that partially applies this law to the channels of the microfluidic chip 10 for the purpose of PCR. When a sample flows through the first temperature region 46 and the second temperature region 48, braking is applied to the sample, adding a function of trying to stop the sample within the first temperature region 46 and the second temperature region 48.

In the present embodiment, as shown in Fig. 3, a part of the first meandering channel 54 and a part of the first widened channel 60 may be included in the first temperature region 46. On the other hand, the second meandering channel 56 and the second widened channel 62 may be included in the second temperature region 48.

The microfluidic chip 10 according to the present embodiment may have at least one sample introduction port for introducing a sample into a channel, and the sample introduction port is, for example, an inlet for introducing a sample containing nucleic acid to be amplified into the channel. The sample introduction port may be of any shape, size, or arrangement, as long as the sample introduction port has a structure perforated from the principal surface of the substrate 14 through one of the channels. In Figs. 3 and 4, as an example, the sample introduction port 44 is provided to connect to a channel belonging to the third meandering channel region 52. In Figs. 3 and 4, the channel 12 is provided on the surface 14a and the sample introduction port 44 is provided on the surface 14b in such a manner that the channel 12 and the sample introduction port 44 are formed on opposite sides of the substrate 14 from each other but may be provided on the same surface of the substrate 14. The sample introduction port 44 may connect to the channel belonging to the third meandering channel region 52 via a channel branched from the channel belonging to the third meandering channel region 52, or may have a perforation structure that allows direct access to the channel belonging to the third meandering channel region 52 (for example, the sample introduction port 44 overlaps with the channel belonging to the third meandering channel region 52 in a plan view). The microfluidic chip 10 may include two sample introduction ports (a first sample introduction port and a second sample introduction port). The third meandering channel region 52 can be used as a temporary sample standby channel used when the microfluidic chip 10 is introduced into the PCR device after a predetermined amount of a sample is introduced through the sample introduction port 44.

As shown in Fig. 1, at least the first sealing film 16 for sealing a channel is attached to the first surface 14a. The first sealing film 16 may be large enough to cover substantially the entire first surface 14a of the substrate 14. In order to connect a nozzle leading from a discharge port of the pump of the liquid delivery system, holes may be created in advance in the first sealing film 16 at respective positions corresponding to the air coupling ports 24 and 26 such that the air coupling ports 24 and 26 are not sealed. The first sealing film 16 may be in a form where the air coupling ports 24 and 26 are sealed. In this case, since a nozzle leading from the discharge port of the pump used in the liquid delivery system for pressurization and air feeding inside the channel is attached to the air coupling ports 24 and 26 when the microfluidic chip 10 is used, if the nozzle end is equipped with a hollow needle or the like, the film can be perforated with the tip of the needle, and the output port of the pump can then be connected to the air coupling ports 24 and 26.

Along with the use of the first sealing film 16 for sealing the channel 12, the filter spaces 36 and 38, and the chambers 32 and 34, excluding the air coupling ports 24 and 26, the second sealing film 18, which is adhesive, may be attached to the first surface 14a of the substrate 14, for example, such that only the second air coupling port 26 is sealed. In this case, the sealing film for sealing does not need to be attached to the first air coupling port 24. When a sample is introduced into the microfluidic chip 10 from the sample introduction port 44, the air coupling port 24 acts as an exhaust port for air corresponding to the volume of the sample introduced into the channel. Then, the output end of the pump of the liquid delivery system may be directly connected to the air coupling ports 24 and 26 of the microfluidic chip 10 in which the sample has been introduced into the channel. The second sealing film may be peeled off at this time. During distribution, although the first air coupling port 24 is open, since the second air coupling port 26 and the other ports are sealed by the sealing films, dust, dirt, dust, etc., are not likely to enter through the first air coupling port 24, allowing for the suppression of contamination.

As shown in Fig. 2, the second surface 14b of the substrate 14 is provided with the groove-shaped air ducts 40 and 42 that connect the air coupling ports 24 and 26 and the filter spaces 36 and 38, and a sample introduction port 44 for introducing a sample into the channel 12. The third sealing film 20 for sealing the groove-shaped air ducts 40 and 42 may be attached to the second surface 14b of the substrate 14. Further, a fourth sealing film for sealing the sample introduction port 44 may be attached to the second surface 14b of the substrate 14. If the air ducts 40 and 42 and the sample introduction port 44 are positioned close to each other, the air ducts 40 and 42 and the sample introduction port 44 may be sealed at the same time by the third sealing film 20. In this case, the fourth sealing film is not necessary.

Figs. 1 and 2 show an example in which the substrate 14 and these sealing films 16, 18, and 20 are attached and integrated. Fig. 2 shows an example of an aspect of the third sealing film 20 that seals the air ducts 40 and 42 and the sample introduction port 44 at the same time.

An example of introducing a sample into the channel via the sample introduction port 44 will be explained. First, the operator peels off the third sealing film 20 and opens the sample introduction port 44. At this time, the third sealing film 20 is peeled off just enough to open the sample introduction port 44, and the grooved air ducts 40 and 42 are left sealed.

Next, the measured sample is placed into the channel 12 through the sample introduction port 44 by a pipette, a dropper, a syringe, or the like. The pipette, the dropper, the syringe, or the like may be further operated to push the sample until the sample reaches the first temperature region 46 or the second temperature region 48 in the thermal cycle region. In this case, the position reached by the sample can be used as the initial position of the sample during PCR or a thermal cycle. The introduced sample pushes air of approximately the same volume as the volume of the sample inside the channel, and the air is released from the first air coupling port 24 that is open. In this case, the first air coupling port 24 serves as an exhaust port during the sample introduction.

Next, the third sealing film 20 is reattached back to the original position. The third sealing film 20 preferably has a property of being able to withstand multiple attachment and peeling-off cycles. This is preferable from the viewpoints of maintaining the cleanliness of the microfluidic chip 10 during distribution and convenience of the sample introduction operation.

The second sealing film 18 and the third sealing film 20 may be provided with tabs 18a and 20a having non-adhesive surfaces on both sides at the ends of the films so that a part of the films can be easily peeled off (and potentially reattached) during use, after being initially attached to the principal surface of the substrate 14. This helps to improve the convenience of the operator in opening the sample introduction port 44 and the air coupling ports 24 and 26 of the microfluidic chip 10.

The third sealing film 20 may be provided with a corner 20b between the sample introduction port 44 and the air duct 42, as shown in Fig. 2. This corner 20b serves as a guide for the limit to which the operator can peel off the third sealing film 20 when opening only the sample introduction port 44. Also, the film may be made less likely to tear by providing a substantially circular cutout in the corner to emphasize the guide or by peeling off the film halfway.

The method for the introduction of a sample to the sample introduction port 44 is not particularly limited. For example, an appropriate amount of the sample may be directly introduced through the sample introduction port 44 using a pipette, a dropper, a syringe, or the like. Alternatively, a method of introduction may be used that is performed while preventing contamination via a needle chip, in which a filter made of porous PTFE or polyethylene is incorporated, or via a syringe filter. In general, many types of such needle chips are sold and can be obtained easily, and the needle chips can be used while being attached to the tip of various pipettes, droppers, syringes, or the like. Furthermore, the sample may be moved to a predetermined position in the channel 12 by discharging and introducing the sample by a pipette, a dropper, a syringe, or the like and then further pushing the sample through pressurization. Although not shown, a plurality of sample introduction ports may be provided. In this case, by using an area of the channel belonging to the third meandering channel region between the plurality of sample introduction ports, it is possible to provide a function capable of easily measuring a sample of a fixed volume that is to be subjected to a reaction process such as PCR (dispensing function). For the detailed method used at this time, the matters described in Japanese Patent Application Publication No. 2016-19606 can be referred to. A channel with such a function of allowing a sample that is to be subjected to PCR to be in a fixed volume may be referred to as a dispensing channel.

The sample includes, for example, those obtained by adding a thermostable enzyme and four types of deoxyribonucleoside triphosphates (dATP, dCTP, dGTP, dTTP) as PCR reagents to a mixture containing one or more types of DNA. Further, a primer that specifically reacts with the DNA subjected to reaction processing, and in some cases, a fluorescent probe such as TaqMan (TaqMan is a registered trademark of Roche Diagnostics Gesellschaft mit beschränkter Haftung) or SYBR Green (SYBR is a registered trademark of Molecular Probes, Incorporated) are mixed. Commercially available real-time PCR reagent kits and the like can be also used.

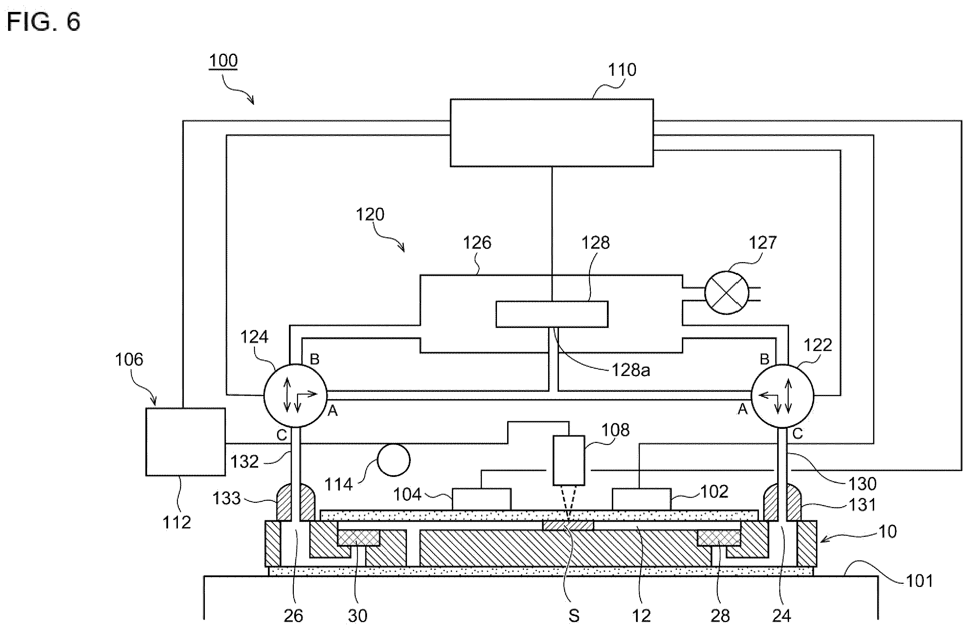

Fig. 6 is a schematic diagram for explaining a PCR device (also referred to as a "thermal cycling device," a "thermal cycler," or a "reaction processor") 100 for performing a thermal cycle and PCR in which a microfluidic chip 10 can be used.

The PCR device 100 includes a temperature control system including an installation unit 101 in which the microfluidic chip 10 is installed, a first heater 102 for heating the first temperature region 46 of the channel 12 that is controlled at a relatively high temperature, a second heater 104 for heating the second temperature region 48 of the channel, and a temperature sensor (not shown) such as, for example, a thermocouple or the like for measuring the actual temperature of each reaction region. Each heater may be, for example, a resistance heating element, a Peltier element, or the like. By these heaters, a suitable heater driver, and a control device 110 including a microcomputer, a microprocessor, an electronic circuit, and the like, the first temperature region 46 in the channel 12 of the microfluidic chip 10 is maintained to be approximately 95°C, and the second temperature region 48 is maintained to be approximately 62°C. Thus, the temperature of each reaction region in a thermal cycle region that is necessary for performing PCR is set. Furthermore, in order to operate each of these parts appropriately, the PCR device 100 is equipped with a battery built inside the device and a power supply unit for receiving power from an external source (neither of which is shown). The explanation and description of drivers for driving each of the parts are omitted.

The PCR device 100 further include a fluorescence detector 106. As described above, a predetermined fluorescent probe is added to a sample S. Since the intensity of a fluorescence signal emitted from the sample S increases as the amplification of the DNA proceeds, the intensity value of the fluorescence signal can be used as an index serving as a decision material for the progress of the PCR or the termination of the reaction.

As the fluorescence detector 106, an optical fiber-type fluorescence detector FLE-510 manufactured by Nippon Sheet Glass Co., Ltd., can be used, which is a very compact optical system that allows for rapid measurement and the detection of fluorescence regardless of whether the place is a lighted place or a dark place. This optical fiber-type fluorescence detector allows the wavelength characteristic of the excitation light/fluorescence to be tuned such that the wavelength characteristic is suitable for the characteristic of fluorescence emitted from the sample S and thus allows an optimum optical and detection system for a sample having various characteristics to be provided. Further, the optical fiber-type fluorescence detector is suitable for detecting fluorescence from a sample existing in a small or narrow region such as a channel because of the small diameter of a ray of light brought by the optical fiber-type fluorescence detector and is also excellent in response speed.

The optical fiber-type fluorescence detector 106 includes an optical head 108, a fluorescence detector main unit (including a driver) 112, and an optical fiber 114 connecting an optical head 80 and the fluorescence detector main unit 82. The fluorescence detector main unit 112 includes a light source for excitation light (LED, a laser, or a light source adjusted to emit other specific wavelengths), an optical fiber-type multiplexer/demultiplexer and a photoelectric conversion device (PD, APD, or a light detector such as a photomultiplier) (neither of which is shown), and the like and formed of a driver, a microcomputer, or the like for controlling these. The optical head 108 is formed of an optical system such as a lens and has a function of directionally irradiating the sample with excitation light and collecting fluorescence emitted from the sample. The collected fluorescence is separated from the excitation light by the optical fiber-type multiplexer/demultiplexer inside the fluorescence detector driver through the optical fiber 114 and converted into an electric signal by the photoelectric conversion element. As the optical fiber-type fluorescence detector, those described in Japanese Patent Application Publication No. 2010-271060 can be used. The optical fiber-type fluorescence detector 106 can be further modified so as to allow for coaxial detection for a plurality of wavelengths using a single or a plurality of optical heads. The invention described in WO 2014/003714 can be used for a fluorescence detector for a plurality of wavelengths and signal processing thereof.

In the PCR device according to the present embodiment, the optical head 108 is arranged such that fluorescence from the sample S in the detection channel 59 can be detected. Since the reaction progresses while the sample S is repeatedly moved in a reciprocating motion in the thermal cycle region inside the channel such that predetermined DNA contained in the sample S is amplified, by monitoring a change in the amount of detected fluorescence, the progress of the DNA amplification can be learned in real time. Further, in the PCR device 100, an output value from the fluorescence detector 106 is utilized for controlling the movement of the sample S. For example, an output value from the fluorescence detector 106 may be transmitted to a control device 110 and may be used as a parameter at the time of controlling a liquid feeding system 120 described later. The fluorescence detector 106 is not limited to an optical fiber-type fluorescence detector as long as the fluorescence detector exhibits the function of detecting fluorescence from a sample.

The fluorescence detector 106 can also provide a plurality of sets of excitation light/fluorescence. For example, a first fluorescence detector may be set to emit blue excitation light and detect green fluorescence emitted from the sample, and a second fluorescence detector may be set to emit green excitation light and detect red fluorescence. In addition, fluorescence detectors may be arranged that are preset for the combination of excitation light/fluorescence wavelength characteristics according to the combination of nucleic acids and fluorescent probes that are to be detected.

In the PCR device 100 according to the present embodiment, the optical head 108 of the fluorescence detector 106 is arranged to detect fluorescence from a sample in the connection channel 50 in the thermal cycle region. The connection channel 50 is a channel connecting the channel included in the first temperature region 46 and the channel included in the second temperature region 48. As described above, the region in which fluorescence from the sample is intended to be detected is referred to as a fluorescence detection region 58. The channel belonging to the fluorescence detection region 58 is referred to as a detection channel 59. The detection channel 59 is provided on the first surface 14a of the substrate 14 where the channel is formed.

In this case, the surface roughness of the region corresponding to the detection region on a surface facing the surface of the substrate 14 on which the detection channel 59 is provided (i.e., the second surface 14b) may be Ra0 = 4 nm to 80 nm, expressed as arithmetic mean roughness. Excitation light emitted from the optical head 108 toward the sample in the detection channel 59 partially passes through the detection channel 59 and reaches the second surface 14b of the opposing substrate 14. A part of the excitation light reaching the second surface 14b of the substrate 14 is subjected to optical effects such as reflection, scattering, and refraction by the second surface 14b. The part of the excitation light that penetrates the channel and reaches the second surface 14b is a part that corresponds to the fluorescence detection region 58. Therefore, if the surface roughness of the part corresponding to the fluorescence detection region 58 on the second surface 14b is large, diffuse reflection and scattering occur, causing a part of the light to return to the optical head 108 side. For example, when employing the second fluorescence detector whose excitation light includes light belonging to a green color as described above, some wavelengths may overlap with that of the fluorescence to be detected by the first fluorescence detector. In such a case, there is a possibility that a part of the excitation light from the second fluorescence detector may be mixed with fluorescence to be detected by the first fluorescence detector due to diffuse reflection or scattering (backscattering). Such interference between the excitation light and the fluorescence from different fluorescence detectors may result in noise in the detected fluorescence. Therefore, for the purpose of suppressing such unexpected return light such as diffuse reflection and scattering, the surface roughness in the region corresponding to the fluorescence detection region 58 on a surface facing the surface of the substrate 14 on which the detection channel 59 is provided may be Ra₀ = 5 nm to 80 nm, expressed as arithmetic mean roughness. It is more preferable that the surface roughness in the region is Ra₀ = 5 nm to 25 nm, expressed as arithmetic mean roughness. The arithmetic mean roughness can be measured using a VK-X3000 laser microscope manufactured by KEYENCE CORPORATION. In this case, given that the magnification of the objective lens is 20x and that the reference length is 100 µm and the number of reference lengths is 5 at least in the region overlapping the channel, the maximum value of Ra that is calculated may be employed as Ra0.

The PCR device 100 is further provided with a liquid feeding system 120 for moving and stopping the sample S inside the channel 12 of the microfluidic chip 10. Although the liquid feeding system 120 is not limited to this, the sample S can be moved in one direction inside the channel by sending (air feeding) the air from one of the first air coupling port 24 and the second air coupling port 26 through the first air coupling port 24 or the second air coupling port 26. Further, the liquid feeding system 120 can be stopped at a predetermined position by stopping the air supply to the channel or by equalizing the pressure on both sides of the sample S inside the channel.

As shown in Fig. 6, the liquid feeding system 120 in the present embodiment includes a liquid feeding pump 128, a first three-way valve 122, a second three-way valve 124, and a pressurizing chamber 126. Further, the liquid feeding system 120 may be equipped with tubes and nozzles that connect the discharge port of the liquid feeding pump 128 and the air coupling ports.

The pressurizing chamber 126 forms a space having a certain volume therein. The pressurizing chamber 126 is formed to be able to change the pressure in an internal space thereof. The pressure inside the pressurizing chamber 126 is maintained at a pressure at least higher than the atmospheric pressure of the environment surrounding the PCR device 100 (e.g., 1.05 to 1.3 atm) while PCR is being performed. In order to increase the pressure within the pressurizing chamber 126, the PCR device 100 may include, but not limited to, a pressurizing pump, an air pump, a blower, a micropump, a syringe pump, or the like (not shown) having an output port connected to the inside of the pressurizing chamber 126. The atmospheric pressure in the environment surrounding the PCR device 100 means the pressure (or atmospheric pressure) at a place where the PCR device 100 is installed, a place where the PCR is performed by the PCR device 100, or, if the PCR device 100 is installed at a place that is divided from the surroundings, the divided place. The pressure inside the pressurizing chamber 126 needs to be applied to such an extent that significant evaporation of the sample and generation of air bubbles or the like, which affect the reaction process involving PCR, can be prevented even when the sample is repeatedly exposed to a high temperature (about 95°C). The higher the pressure inside the pressurizing chamber 126 becomes, the more the influence of the evaporation of the sample and the like can be suppressed. However, on the other hand, the liquid feeding system 120 becomes complicated or enlarged including the handling thereof. Thus, a person skilled in the art can comprehensively judge the application, purpose, cost, effect, etc., of the processor so as to design the entire system.

An atmospheric pressure releasing valve (leak valve) 127 is provided in the pressurizing chamber 126. The atmospheric pressure releasing valve 127 is controlled such that the pressure of the liquid feeding system 120 and the pressure of the microfluidic chip 10 in the channel 12 become equal to the atmospheric pressure at the time of installing or removing the microfluidic chip 10. Thereby, rapid movement and squirting of the sample S can be prevented.

The liquid feeding pump 128 is arranged inside the pressurizing chamber 126. The liquid feeding pump 128 may be a blower or microblower that allows the pressure on the primary side (air suction side) and the pressure on the secondary side (air discharge side) to become equal when stopped. It should be noted that the liquid feeding pump 128 has the role of sending air into the channel such that a liquid sample moves in response to the air feeding and increased pressure and is not a pump that actually pumps the liquid itself.

A first port A of the first three-way valve 122 is connected to an output port (discharge port) 128a of the liquid feeding pump 128. A second port B of the first three-way valve 122 is connected to the interior space of the pressurizing chamber 126. A third port C of the first three-way valve 122 is connected to one end of the first tube 130, and the other end of the first tube 130 is connected to the first air coupling port 24 of the microfluidic chip 10 as the first output port 131 of the liquid feeding system 120. The first three-way valve 122 can be switched between a state in which the first air coupling port 24 of the microfluidic chip 10 connects to the output port 128a of the liquid feeding pump 128 and a state in which the first air coupling port 24 of the microfluidic chip10 is open to the internal space of the pressurizing chamber 126. When the first air coupling port 24 of the microfluidic chip 10 connects with the output port 128a of the liquid feeding pump 128, the first three-way valve 122 is controlled such that the port A and the port C connect to each other. On the other hand, when the first air coupling port 24 of the microfluidic chip 10 is opened to the internal space of the pressurizing chamber 126, the first three-way valve 122 is controlled such that the port B and the port C connect to each other.

A first port A of the second three-way valve 124 is connected to an output port 128a of the liquid feeding pump 128. A second port B of the second three-way valve 124 is connected to the interior space of the pressurizing chamber 126. A third port C of the second three-way valve 124 is connected to the second air coupling port 26 of the microfluidic chip 10. The third port C of the second three-way valve 124 is connected to one end of the second tube 132, and the other end of the second tube 132 is connected to the second air coupling port 26 of the microfluidic chip 10 as the second output port 133 of the liquid feeding system 120. The second three-way valve 124 can be switched between a state in which the second air coupling port 26 of the microfluidic chip10 connects with the output port 128a of the liquid feeding pump 128 and a state in which the second air coupling port 26 of the microfluidic chip10 is open to the internal space of the pressurizing chamber 126. When the second air coupling port 26 of the microfluidic chip 10 connects with the output port 128a of the liquid feeding pump 128, the second three-way valve 124 is controlled such that the port A and the port C connect with each other. On the other hand, when the second air coupling port 26 of the microfluidic chip 10 is opened to the internal space of the pressurizing chamber 126, the second three-way valve 124 is controlled such that the port B and the port C connect to each other.

The operation of the liquid feeding pump 128, the first three-way valve 122, and the second three-way valve 124 can be performed by the control device 110 through an appropriate driver. In particular, the optical head 108 arranged as described above can control the liquid feeding system 120 by transmitting an output value that is based on an obtained fluorescence signal to the control device 110 and causing the control device 110 to recognize the position and passage of the sample S in the channel.

An explanation will be given regarding a case where the sample S is moved from the first temperature region 46 to the second temperature region 48 using the liquid feeding system 120. The liquid feeding pump 128 is controlled to be in an operating state (ON). Further, the first three-way valve 122 is controlled such that the first port A and the third port C connect to each other, and the second three-way valve 124 is controlled such that the second port B and the third port C connect to each other. Thereby, the first air coupling port 24 of the microfluidic chip10 connects to with the output port 128a of the liquid feeding pump 128, and the second air coupling port 26 of the microfluidic chip10 is opened to the internal space of the pressurizing chamber 126. In the present embodiment, since the liquid feeding pump 128 is arranged inside the pressurizing chamber 126, when air is discharged from the liquid feeding pump 128, the pressure at the first air coupling port 24 of the microfluidic chip 10 becomes higher than that at the second air coupling port 26, and the sample S thus moves from the first temperature region 46 to the second temperature region 48. The first three-way valve 122 is controlled such that the second port B and the third port C connect to each other, and the second three-way valve 124 is controlled such that the second port B and the third port C connect to each other, when the sample S reaches the second temperature region 48. Thereby, both the first air coupling port 24 and the second air coupling port 26 are opened to the internal space of the pressurizing chamber 126, and the sample S thus stops in the second temperature region 48. Conversely, when the sample S is to be moved from the second temperature region 48 to the first temperature region 46, the first three-way valve 122 and the second three-way valve 124 need to be operated in the reverse manner of the way described above.

As described, by sequentially and continuously operating the first three-way valve 122 and the second three-way valve 124 connected to the respective sides of the channel 12, the sample S is continuously moved in a reciprocating motion between the first temperature region 46 and the second temperature region 48 in the channel. This allows an appropriate thermal cycle to be applied to the sample **S.**

In the PCR device 100 according to the present embodiment, the liquid feeding pump 128 is arranged in the internal space of the pressurizing chamber 126 set to a pressure higher than the pressure of the surrounding environment (for example, 1.3 atm), and the second port B of the first three-way valve 122 and the second port B of the second three-way valve 124 are configured to be opened to the internal space of the pressurizing chamber 126. Therefore, during the reaction process, the entire channel 12 of the microfluidic chip 10 is maintained at a pressure higher than the atmospheric pressure of the surrounding environment. This is particularly beneficial when using the PCR device 100 at high altitudes, for example, in Mexico City or in an aircraft in flight. The first temperature in the first temperature region 46 is at or close to 95°C. Since the pressure at high altitudes and in an aircraft is less than 1 atm, there is a possibility that a part of the sample S boils even at 95°C. However, by employing the liquid feeding system 120 provided with the pressurizing chamber 126 of the PCR device 100, it is possible to suppress undesirable sample boiling in such a low atmospheric pressure environment. Further, the PCR device 100 according to the present embodiment can also be used without problems even in a low atmospheric pressure environment.

For the liquid feeding system, a form other than the form shown in Fig. 6 may be employed. Another form of the liquid feeding system will be described later. Syringe pumps, diaphragm pumps, microblowers, blowers, and the like can be used for pumps. Since two pumps are used, it may be necessary to adjust individual differences between the pumps in order for accurate stopping in a desired temperature range. In this case, air feeding pumps consisting of blowers or microblowers can be also used that allow the pressure on the primary side (air suction side) and the pressure on the secondary side (air discharge side) to become equal when stopped, for the two pumps. When moving the sample, only the pump corresponding to that direction is operated. When stopping the sample, both of the pumps are stopped. This balances the pressure on both sides of the sample such that the sample can be stopped.

As an exemplary variation, in the PCR device 100 shown in Fig. 6, a filter for contamination prevention may be provided in the first output port 131 and the second output port 133. For example, a syringe tip (syringe needle) or syringe filter with a built-in filter or the like may be used as an example of these output ports. Syringe tips with built-in filters are inexpensive, and are easy to obtain since many types are available on the market. A syringe tip with a built-in filter may be attached to the end of the liquid feeding system from the pump, and the tip may be, e.g., inserted into the air coupling port of the microfluidic chip 10 and fixed thereto, thereby establishing air connection to the channel 12 of the microfluidic chip 10. If greater cleanliness is required, the syringe tip may be replaced for each lot. Further, a pipette tip with a micropipette filter may be used instead of a syringe tip (syringe needle) or syringe filter. The tip of a filter-equipped pipette tip of a micropipette may be connected to the first air coupling port 24 and the second air coupling port 26, and the micropipette may be removed while the pipette tip is connected to the air coupling ports, and the pipette tip may then be connected to the liquid feeding mechanism. In this case, the tip of the filter-equipped pipette tip of the micropipette may be connected to a sample introduction port, and the micropipette may be removed while the pipette tip is connected to the air coupling ports, and the pipette tip may then be connected to the liquid feeding mechanism.

As described, it is not mandatory to include the first filter 28 or the second filter 30 in the microfluidic chip 10 if the filtering function is provided on the side of the liquid feeding system 120 included in the PCR device 100. In this case, the structure of the substrate 14 of the microfluidic chip 10 may be such that the first chamber 32 directly connects to the first air coupling port 24 or that the second chamber 34 connects to the second air coupling port 26 in the same manner.

Further, in the microfluidic chip 10, it is possible to modify the structure so that the first temperature region 46 and the second temperature region 48, each maintaining the temperature required for PCR, are provided along the channel 12 and that the third meandering channel region 52 described above directly contributes to the thermal cycle and PCR serving as a so-called third temperature region. For example, the first temperature region 46 may be maintained at 94°C to 96°C required for the thermal denaturation reaction, the second temperature region 48 may be maintained at 72°C to 74°C required for the elongation reaction, and the third meandering channel region 52 may be transformed to be maintained at 55°C to 60°C required for annealing as the third temperature region. When embodying such an exemplary variation, the temperature control system of the PCR device is further provided with a heater (third heater), a temperature measurement function, a driver, and the like necessary to heat and maintain all or a part of the channel in the third meandering channel region 52.

Such a system that applies a thermal cycle to a sample by means of regions maintained at three different temperatures may be referred to as a "three-temperature system" (the system explained in the previous embodiment may be referred to as a "two-temperature system"). In the case of a three-temperature system, rather than a simple reciprocating movement as in a two-temperature system, it is necessary to repeat a cycle of, for example, the first temperature zone (denaturation) → the third temperature zone (annealing) → the second temperature zone (elongation), making the system somewhat more complicated than a two-temperature system. However, compared to a two-temperature system in which annealing and elongation are performed within the same temperature range, this system has the advantage of providing more options for primers, etc., to be added to a sample.

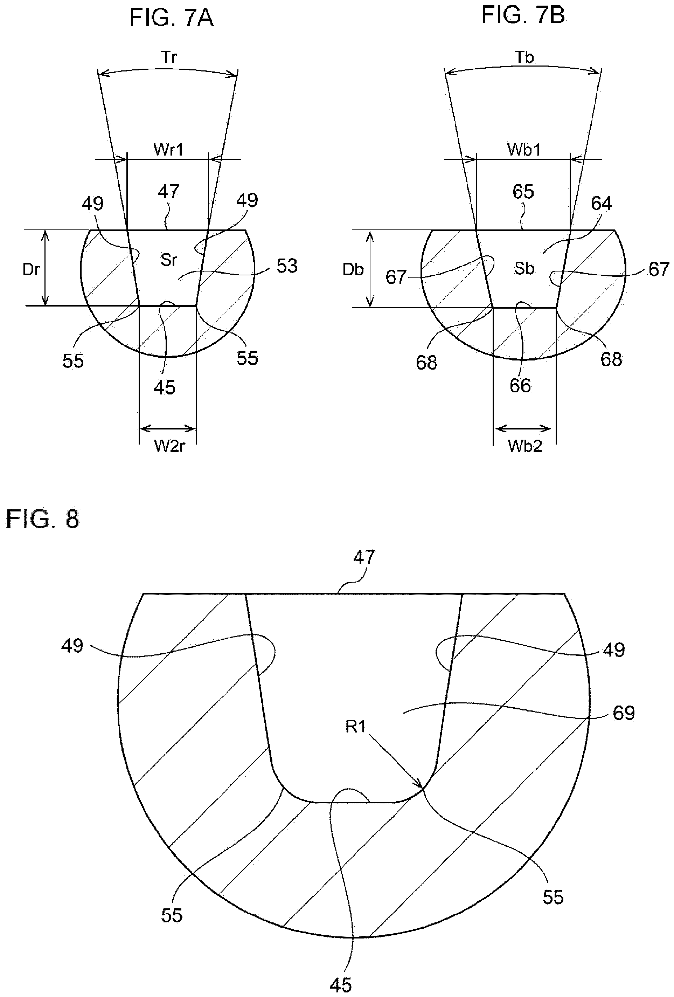

Figs. 7A and 7B are diagrams for explaining the shape of a channel in the substrate 14 of the microfluidic chip 10. The meandering channel 53 with an aspect represented in the cross-sectional view of Fig. 7A corresponds to the first meandering channel 54 and the second meandering channel 56 shown in Fig. 3. A widened channel 64 shown in Fig. 7B corresponds to the first widened channel 60 and the second widened channel 62 shown in Fig. 3.

As shown in Fig. 7A, the meandering channel 53 is a trapezoidal channel and has an opening 47, a bottom surface 45, and side surfaces 49 located on the respective sides of the bottom surface 45. The side surfaces 49 are formed in a tapered shape expanding from the bottom surface 45 toward the opening 47. Parameters defining the shape and dimensions of the meandering channel 53 include a depth Dr, a taper angle Tr, an opening width Wr1, a bottom surface width Wr2, and a cross-sectional area Sr. The bottom surface 45 and the side surfaces 49 have a planar shape, and connections 55 between the bottom surface 45 and the side surfaces 49 have an angular shape, but may be formed to include a curved surface.

As shown in Fig. 7B, the widened channel 64 is also a trapezoidal channel and has an opening 65, a bottom surface 66, and side surfaces 67 located on the respective sides of the bottom surface 66. The side surfaces 67 are formed in a tapered shape expanding from the bottom surface 66 toward the opening 65. Parameters defining the shape and dimensions of the widened channel 64 include a depth Db, a taper angle Tb, an opening width Wb1, a bottom surface width Wb2, and a cross-sectional area Sb. The bottom surface 66 and the side surfaces 67 have a planar shape, and connections 68 between the bottom surface 66 and the side surfaces 67 are an angular shape, but may be formed to include a curved surface.

In the microfluidic chip 10, the cross-sectional area Sb of the widened channel 64 is larger than the cross-sectional area Sr of the meandering channel 53. The moving speed of the sample varies depending on the cross-sectional area of the channel. In general, as the cross-sectional area of the channel increases, the moving speed of the sample decreases. By making the cross-sectional area Sb of the widened channel 64 larger than the cross-sectional area Sr of the meandering channel 53, a braking action is generated on the sample. Thus, the movement/stop control of the sample by the liquid feeding system 120 of the PCR device 100 becomes easy, and the accuracy of stopping the sample at a predetermined position in the meandering channel 53 can be improved. Further, even when the sample is introduced into the channel 12 in an amount larger than a predetermined amount, an excessive overrun of the sample from the meandering channel 53 can be suppressed.

The cross-sectional area ratio Sb/Sr of the cross-sectional area Sr of the meandering channel 53 and the cross-sectional area Sb of the widened channel 64 may be in a range of 1 < Sb/Sr ≤ 1.8, preferably in a range of 1.02 ≤ Sb/Sr ≤ 1.5, and more preferably in a range of 1.02 ≤ Sb/Sr ≤ 1.2. By setting the cross-sectional area ratio Sb/Sr to a value within such a range, the above-described braking action can be suitably generated.

In the microfluidic chip 10, the opening width Wr1 of the meandering channel 53 may be 0.55 mm to 0.95 mm, and preferably 0.65 mm to 0.85 mm. The bottom surface width Wr2 of the meandering channel 53 may be 0 mm to 0.95 mm, and preferably 0.4 mm to 0.6 mm. The depth Dr of the meandering channel 53 may be 0.5 mm to 0.9 mm, and preferably 0.6 mm to 0.8 mm. The taper angle Tr of the meandering channel 53 may be 0° to 45°, preferably 10° to 30°, and more preferably 15° to 25°. It should be noted that in the microfluidic chip 10, the cross-sectional shape of the meandering channel 53 becomes close to a substantially inverse triangular shape when the bottom surface width Wr2 of the meandering channel 53 is smaller than the opening width Wr1 such that the bottom surface width Wr2 is 0 mm or around 0 mm. Further, it should be noted that when the taper angle Tr is reduced to be 0° or around 0°, the cross-sectional shape of the meandering channel 53 becomes close to a substantially rectangular shape.

The opening width Wb1 of the widened channel 64 may be 0.65 mm to 1.05 mm, and preferably 0.75 mm to 0.95 mm. The bottom surface width Wb2 of the widened channel 64 may be 0 mm to 1.05 mm, and preferably 0.5 mm to 0.7 mm. The depth Db of the widened channel 64 may be 0.5 mm to 0.9 mm, and preferably 0.6 mm to 0.8 mm. The taper angle Tb of the widened channel 64 may be 0° to 45°, and preferably 10° to 35°. It should be noted that in the microfluidic chip 10, the cross-sectional shape of the widened channel 64 becomes close to a substantially inverse triangular shape when the bottom surface width Wb2 of the widened channel 64 is smaller than the opening width Wb1 such that the bottom surface width Wb2 is 0 mm or around 0 mm. Further, it should be noted that when the taper angle Tb is reduced to be 0° or around 0°, the cross-sectional shape of the widened channel 64 becomes close to a substantially rectangular shape.

By forming the meandering channel 53 and the widened channel 64 with the dimensions described above, the continuous movement of the sample (which may include a surfactant) becomes smooth, allowing for easy production also by a conventional manufacturing technique such as injection molding.

Fig. 8 shows an exemplary variation of a meandering channel. In the meandering channel 53 shown in Fig. 7A, the connections between the bottom surface 45 and the side surfaces 49 are angular. However, in the meandering channel 69 shown in Fig. 8, connections 55 between the bottom surface 45 and the side surfaces 49 have a curved surface. In Fig. 8, the bottom surface 45 has a planar shape, but may have a curved surface continuously connected to the connections 55.

In the present embodiment, as described above, a meandering channel is employed for the purpose of improving the efficiency of heating by a heater. However, depending on an injection molding method in which a mold is filled with a resin at a high speed, there are some cases when the shape of such a channel becomes a resistance or an obstacle, and molding may not be performed properly, for example, causing a variation in the speed of filling with the resin or the generation of a void. In such a case, a weld line may be formed in a region including the meandering channel, and a recess such as a pit may be formed on the channel. Such a recess may obstruct the flow of the sample. As in the meandering channel 56 according to the present exemplary variation, by forming the connections 55 to have a curved surface, the flow of the resin inside the mold at the time of injection molding becomes smooth. Thus, the generation of a weld line near a reaction channel can be suppressed. The curvature radius R1 of the connections 55 may be, for example, 0.2 mm to 0.38 mm, and preferably 0.3 mm to 0.35 mm. In the same manner, in the widened channel 64, a connection 68 between the bottom surface 66 and the side surfaces 67 may have a curved surface.

As shown in Fig. 3, the first meandering channel 54 and the second meandering channel 56 include a plurality of curved channels. The curvature radius R2 of the curved channels may be, for example, 0.3 mm to 10 mm, and preferably 0.5 mm to 6 mm. By setting the curvature radius of the meandering channels to be within such a range, the generation of a weld line can be further suppressed, and furthermore, when the sample moves inside the meandering channel, the moving speed of the sample can be easily kept constant in the meandering channels.

As explained with reference to Fig. 6, the PCR device 100 includes a fluorescence detector 106 in order to irradiate a sample moving inside the channel of the microfluidic chip with excitation light during the PCR and measure the fluorescence emitted from the sample. In the fluorescence detector 106, the optical head 108 has a function of directionally irradiating the sample with excitation light and collecting fluorescence emitted from the sample. The focused spot diameter of the optical head 108 is usually about 0.15 mm to 0.45 mm, which is extremely small. Therefore, when arranging and fixing the optical head 108 to the PCR device 100, very high accuracy is required. As a result, the workability for assembling may be reduced, and the cost for the parts may be increased. Therefore, in another embodiment of the present invention, a microfluidic chip is provided that can overcome such a problem.

A microfluidic chip according to the other embodiment of the present invention also includes a substrate, a sealing film attached to the substrate, and a filter. The same components as those of the microfluidic chip 10 are denoted by the same reference numerals, and redundant description will be omitted as appropriate.

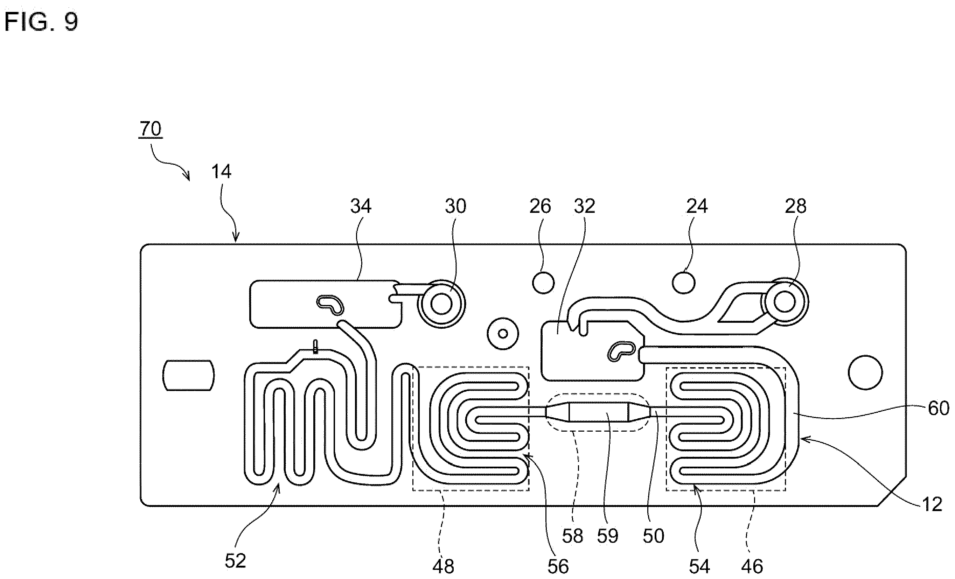

Fig. 9 is a plan view of a substrate 14 provided in a microfluidic chip 70 according to another embodiment of the present invention. The microfluidic chip 70 has a configuration different from that of the microfluidic chip 10 with respect to the detection channel 59.

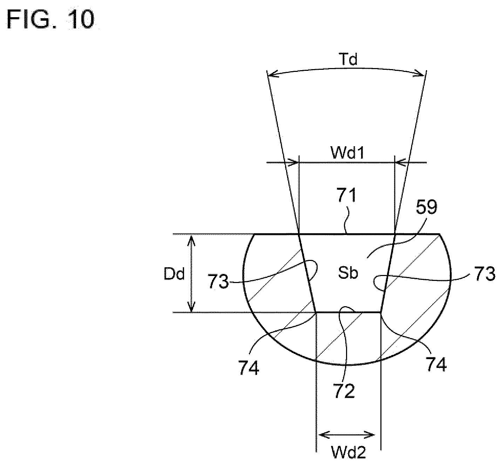

Fig. 10 shows a cross-section of the detection channel 59 of the microfluidic chip 70. The detection channel 59 is provided in the connection channel 50 and is irradiated with excitation light in order to detect fluorescence from a sample. As shown in Fig. 10, the detection channel 59 is a trapezoidal channel and includes an opening 71, a bottom surface 72, and side surfaces 73 located on the respective sides of the bottom surface 72. The side surfaces 73 are formed in a tapered shape expanding from the bottom surface 72 toward the opening 71. Parameters defining the shape and dimensions of the detection channel 59 include a depth Dd, a taper angle Td, an opening width Wd1, a bottom surface width Wd2, and a cross-sectional area Sd.

In the microfluidic chip 70, the cross-sectional area Sr of the meandering channel 53 (see Fig. 7A) is larger than the cross-sectional area Sd of the detection channel 59. By making the cross-sectional area Sd of the detection channel 59 larger than the cross-sectional area Sr of the meandering channel 53 as described, the tolerance when assembling the optical head 108 to the PCR device 100 is eased, thus improving the workability for assembling and lowering the cost for the parts.

In the microfluidic chip 70, the cross-sectional area ratio Sd/Sr of the cross-sectional area Sr of the meandering channel 53 and the cross-sectional area Sd of the detection channel 59 may be in a range of 1 < Sd/Sr ≤ 1.8, preferably in a range of 1.02 ≤ Sd/Sr ≤ 1.5, and more preferably in a range of 1.02 ≤ Sd/Sr ≤ 1.2.

In the microfluidic chip 70, the opening width Wr1 of the meandering channel 53 may be 0.55 mm to 0.95 mm, and preferably 0.65 mm to 0.85 mm. The bottom surface width Wr2 of the meandering channel 53 may be 0 mm to 0.95 mm, and preferably 0.4 mm to 0.6 mm. The depth Dr of the meandering channel 53 may be 0.5 mm to 0.9 mm, and preferably 0.6 mm to 0.8 mm. The taper angle Tr of the meandering channel 53 may be 0° to 45°, preferably 10° to 30°, and more preferably 15° to 25°. It should be noted that in the microfluidic chip 70, the cross-sectional shape of the meandering channel 53 becomes close to a substantially inverse triangular shape when the bottom surface width Wr2 of the meandering channel 53 is smaller than the opening width Wr1 such that the bottom surface width Wr2 is 0 mm or around 0 mm. Further, it should be noted that when the taper angle Tr is reduced to be 0° or around 0°, the cross-sectional shape of the meandering channel 53 becomes close to a substantially rectangular shape.

In the microfluidic chip 70, the opening width Wd1 of the detection channel 59 may be 0.7 mm to 1,2 mm, and preferably 0.8 mm to 1.1 mm. The bottom surface width Wd2 of the detection channel 59 may be 0.15 mm to 1.2 mm, and preferably 0.55 mm to 0.95 mm. The depth Dd of the detection channel 59 may be 0.5 mm to 1.2 mm, and preferably 0.6 mm to 1.1 mm. The taper angle Td of the detection channel 59 may be 0° to 45°, preferably 10° to 35°, and more preferably 15° to 30°.

By forming the meandering channel 53 and the detection channel 59 with the dimensions described above, the continuous movement of the sample (which may include a surfactant) becomes smooth, allowing for easy production also by a conventional manufacturing technique such as injection molding.

Furthermore, by increasing the bottom surface width Wd2 in the cross section of the detection channel 59, the effect on the above-described tolerance can be further improved, and the distance between the opposing side surfaces 73 is increased. Thereby, the possibility that reflection, refraction, scattering, or the like at the side surfaces 73 hinders stable measurement of fluorescence can be lowered.

The bottom surface 72 of the detection channel 59 is formed on a plane parallel to the principal surface (i.e., the first surface 14a and the second surface 14b) of the substrate 14. Further, when the microfluidic chip 70 is set in the PCR device 100 (see Fig. 6), the optical head 108 of the fluorescence detector 106 is arranged such that the optical axis thereof is substantially perpendicular to the bottom surface 72 and the principal surface of the substrate 14. With such an arrangement, undesirable refraction or reflection of excitation light emitted from the optical head 108 to the sample or fluorescence emitted from the sample can be suppressed, and stable fluorescence intensity detection can be performed.

The bottom surface 72 and the side surfaces 73 have a planar shape, and connections 74 between the bottom surface 72 and the side surfaces 73 have an angular shape. That is, the connections 74 between the bottom surface 72 and the side surfaces 73 substantially have no curved surface, and for example, the approximate curvature radius thereof may be 0.02 mm or less, preferably 0.01 mm or less, and more preferably 0.005 mm or less. In the fluorescence detection region 58, if a curved surface or the like is present in a part corresponding to a fluorescence emission part or an excitation light irradiation part, the curved surface or the like may cause irregular refraction or scattering, which may hinder stable measurement of fluorescence. Therefore, such a situation can be prevented from occurring by making the cross section of the detection channel 59 to have a shape in which a curved surface or the like is eliminated as much as possible.

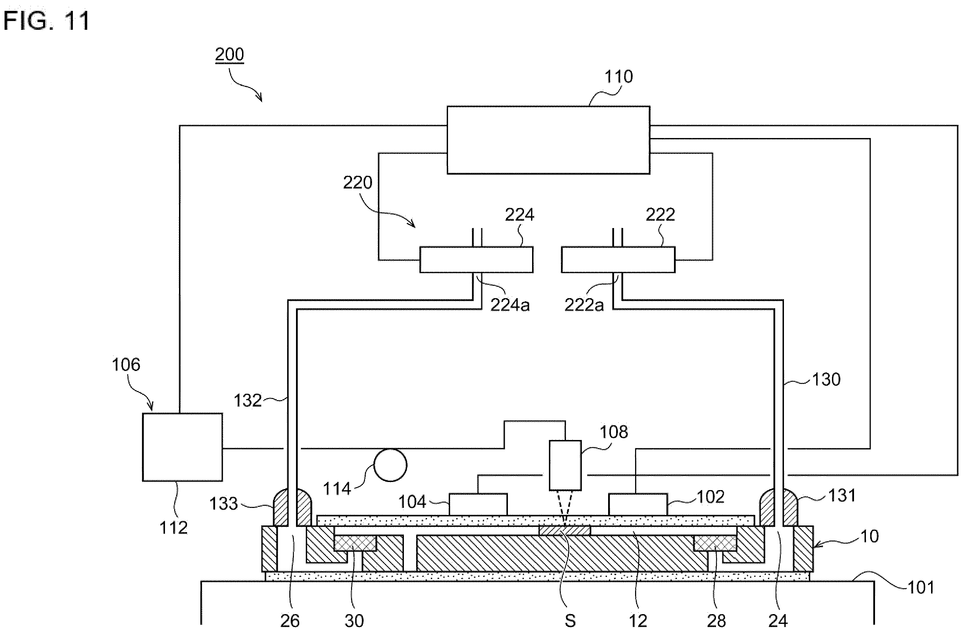

Fig. 11 is a schematic diagram for explaining another embodiment of a PCR device for performing a thermal cycle and PCR in which a microfluidic chip can be used. In the PCR device 200 shown in Fig. 11, the configuration of a liquid feeding system 220 is different from that of the liquid feeding system 120 of the PCR device 100 shown in Fig. 6. The liquid feeding system 220 includes a first liquid feeding pump 222 and a second liquid feeding pump 224. The liquid feeding system 220 does not include a three-way valve. It should be noted that both of the liquid feeding pumps also have the role of sending air into the channel such that a liquid sample moves in response to the air feeding and increased pressure and are not pumps that actually pumps the liquid itself.

In the liquid feeding system 220, the first liquid feeding pump 222 and the second liquid feeding pump 224 are arranged not inside the chamber but in atmospheric pressure. An output port 222a of the first liquid feeding pump 222 connects to the first air coupling port 24 of the microfluidic chip 10 via the first tube 130. An output port 224a of the second liquid feeding pump 224 connects to the second air coupling port 26 of the microfluidic chip 10 via the second tube 132. On the other hand, as in the liquid feeding system 120 of the PCR device 100, the liquid feeding system 220 may be provided with a pressurizing chamber including the first liquid feeding pump, the second liquid feeding pump, and the input ports (suction ports) of the liquid feeding pumps, and, accordingly, may also be provided with an atmospheric pressure release valve (leak valve) and a pump, a blower, a micropump, or the like for increasing the pressure in the pressurizing chamber.

In the liquid feeding system 220 formed and arranged in this manner, alternately operating the first liquid feeding pump 222 and the second liquid feeding pump 224 for discharging causes a sample S to move in a reciprocating motion inside the channel 12 of the microfluidic chip 10, thereby providing the sample S with an appropriate thermal cycle.

Described above is an explanation of the present invention based on the embodiments. These embodiments are intended to be illustrative only, and it will be obvious to those skilled in the art that various modifications to constituting elements and processes could be developed and that such modifications are also within the scope of the present invention.

### INDUSTRIAL APPLICABILITY

The present invention is applicable to a polymerase chain reaction (PCR).

### REFERENCE SIGNS LIST

10, 70 microfluidic chip, 12 channel, 14 substrate, 16 first sealing film, 18 second sealing film, 20 third sealing film, 24 first air coupling port, 26 second air coupling port, 28 first filter, 30 second filter, 32 first chamber, 34 second chamber, 40 first air duct, 42 second air duct, 46 first temperature region, 48 second temperature region, 54 first meandering channel, 56 second meandering channel, 58 fluorescence detection region, 59 detection channel. 60 first widened channel, 62 second widened channel, 100, 200 PCR device, 101 installation unit, 102 first heater, 104 second heater, 106 fluorescence detector, 108 optical head, 110 control device, 120, 220 liquid feeding system, 122 first three-way valve, 124 second three-way valve, 126 pressurizing chamber, 128 liquid feeding pump, 222 first liquid feeding pump, 224 second liquid feeding pump

## Claims

1. A microfluidic chip comprising:
a substrate;
a channel that is formed on the substrate and that is for a sample to move;
air coupling ports that are provided at both ends of the channel;
thermal cycle regions that are provided in a part of the channel and that are for applying a thermal cycle to the sample in the channel; and
a chamber that is provided between the thermal cycle region and the air coupling port in the channel.

2. The microfluidic chip according to Claim 1, wherein given that the volume of the sample that is introduced into the channel is V₀, the volume V₁ of the chamber is expressed as 1.0 × V₀ ≤ V₁ ≤ 6.0 × V₀.

3. The microfluidic chip according to Claim 1 or 2, wherein the volume of the chamber is 25 mm³ to 100 mm³.

4. The microfluidic chip according to any one of Claims 1 to 3, wherein
the chamber includes:
a first connection to a channel leading to the thermal cycle region;
a second connection to a channel leading to the air coupling port; and
a structure that prevents the sample that has entered the chamber through the first connection from heading directly toward the second connection.

5. The microfluidic chip according to Claim 4, wherein the structure is provided such that the structure divides a virtual straight line connecting the first connection and the second connection.

6. The microfluidic chip according to any one of Claims 1 to 5, wherein the chamber has a bottom surface parallel to a principal surface of the substrate.

7. The microfluidic chip according to any one of Claims 1 to 5, wherein the chamber includes a convex structure protruding from the bottom surface.

8. The microfluidic chip according to Claim 4 or 5, wherein the chamber is provided near the first connection.

9. The microfluidic chip according to any one of Claims 1 to 8, wherein the chamber has a gap between the bottom surface of the chamber and the bottom surface of a connection to a channel leading to the air coupling port.

10. The microfluidic chip according to Claim 9, wherein the gap is 0.05 mm to 1 mm.

11. The microfluidic chip according to any one of Claims 1 to 10, wherein
the thermal cycle region includes a meandering channel that includes a straight channel and a curved channel, and
a channel that connects between the meandering channel and the chamber includes a channel formed to have a cross-sectional area larger than the cross-sectional area of the meandering channel.

12. The microfluidic chip according to any one of Claims 1 to 11, wherein
the thermal cycle region includes a first temperature region maintained at a first temperature, a second temperature region maintained at a second temperature different from the first temperature, and a connection channel connecting between the first temperature region and the second temperature region, and
the surface roughness of a region corresponding to the connection channel on a surface facing a surface of the substrate on which the connection channel is provided is expressed to be Ra₀ = 4 nm to 100 nm as arithmetic mean roughness.

13. The microfluidic chip according to any one of Claims 1 to 12, wherein
the thermal cycle region includes a first temperature region maintained at a first temperature and a second temperature region maintained at a second temperature that is different from the first temperature,
the first temperature region and the second temperature region each include a meandering channel that includes a straight channel and a curved channel, and
a distance between the first temperature region and the second temperature region is longer than the straight channel included in the first temperature region and/or the second temperature region.

14. A PCR device comprising:
the microfluidic chip according to any one of Claims 1 to 13;
a temperature control system that is for adjusting the temperature of the thermal cycle region; and
a liquid feeding system that moves and stops a sample in the channel.

15. A PCR method comprising:
introducing a sample into the microfluidic chip according to any one of Claims 1 to 13;
adjusting the temperature of the thermal cycle region; and
applying a thermal cycle to the sample by moving and stopping the sample in the channel.
